# EUROPÄISCHE PATENTSCHRIFT

(11) **EP 1 886 662 B1**
(45) Veröffentlichungstag und Bekanntmachung des Hinweises auf die Patenterteilung: **09.04.2014**
(21) Anmeldenummer: 07110065.5
(22) Anmeldetag: 12.06.2007
(51) Int. Cl.: A61K 8/42, A61Q 11/00, A23G 4/06, A61K 9/00, A61K 8/44, A61K 8/11

(54) **Antimikrobiell wirksame Verbindungen zur Behandlung von Mundgeruch**
Anti-microbial compounds for treating bad breath
Compositions actives anti-microbiennes destinées au traitement de la mauvaise haleine

(30) Priorität: 08.09.2006 US 842999 P; 14.06.2006 WO PCT/EP2006/063175
(43) Veröffentlichungstag der Anmeldung: 13.02.2008
(73) Patentinhaber: Symrise AG, 37603 Holzminden (DE)
(72) Erfinder: Rabenhorst, Jürgen, 37671, Höxter (DE); Machinek, Arnold, 37603, Holzminden (DE); Schmaus, Gerhard, 37671, Höxter-Bosseborn (DE); Herrmann, Martina, 31789, Hameln (DE); Vielhaber, Gabriele, 37603, Holzminden (DE); Pillai, Ravikumar, EMERSON,, NJ 07630 (US)
(74) Vertreter: Eisenführ Speiser

(56) Entgegenhaltungen:
- WO-A-01/51454
- WO-A-2004/047833
- WO-A-2006/134013
- WO-A-2006/134120
- US-A- 3 940 422
- US-A- 5 043 154
- US-A- 5 531 982

## Beschreibung

Die Erfindung betrifft primär bestimmte Verwendungen einer Verbindung der Formel 1 oder von Mischungen aus zwei oder mehr unterschiedlichen Verbindungen der Formel 1, insbesondere zur Herstellung eines antimikrobiell wirkenden Mittels und Mittels gegen Mundgeruch sowie entsprechende Verfahren. Ferner betrifft die Erfindung bestimmte Mundhygieneprodukte, umfassend oder bestehend aus einer Verbindung der Formel 1 oder einer Mischung aus zwei oder mehreren unterschiedlichen Verbindungen der Formel 1 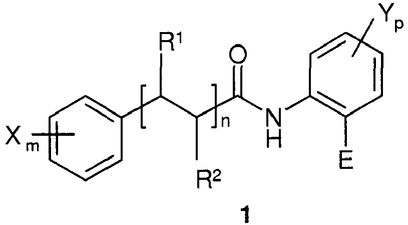 wobei für die Verbindung der Formel 1 bzw. jede Verbindung der Formel 1 in der Mischung gilt:
m = 0, 1, 2 oder 3,
p = 0, 1 oder 2,
n = 0, 1 oder 2, vorzugsweise bedeutet n = 0 oder 1,
wobei bei n = 1 oder 2 jeweils paarweise R¹ und R² jeweils H oder zusammen eine weitere chemische Bindung bedeuten; (wie z.B. in Zimtsäurederivaten),
wobei bei m = 1, 2 oder 3 jedes X, unabhängig von den anderen, OH, OAlkyl oder OAcyl bedeutet,
wobei bei p = 1 oder 2 jedes Y, unabhängig von den anderen, OH, OAlkyl oder OAcyl bedeutet,
wobei E = H oder einen Rest -COOR³ bedeutet,
R³ = H oder Alkyl (insbesondere CH₃, lineare oder verzweigte Alkylketten mit 2 bis 30 C-Atomen), wobei R³ = H auch für die korrespondierenden pharmazeutisch akzeptablen Salze und Solvate steht.

WO 2004/047833 offenbart, dass bestimme Anthranilsäureamide (einer Formel 1) eine Substanz P-induzierte Freisetzung von Histaminen aus Mastzellen inhibieren und somit als kosmetische und pharmazeutische Mittel zur Linderung von Juckreiz geeignet sind. Einige der in der WO 2004/047833 angegebenen Verbindungen der Formel 1 sind auch für die Verwendung im Rahmen der vorliegenden Erfindung besonders bevorzugt.

Die vorliegende Erfindung steht ebenfalls im Zusammenhang mit der eigenen Patentanmeldung PCT/EP 2006/063175, deren kompletter Inhalt im Wege der Verweisung als Bestandteil der vorliegenden Anmeldung inkorporiert ist. PCT/EP 2006/063175 betrifft Mischungen umfassend Anthranilsäureamide der Formel 1 und Kühlwirkstoffe als kosmetische und pharmazeutische Mittel zur Linderung von Juckreiz.

Die gesunde menschliche Schleimhaut des Mund- und Rachenraums sowie die feste Zahnsubstanz ist mit einer Vielzahl nicht pathogener Mikroorganismen besiedelt. Diese sogenannte Mikroflora der Mundhöhle ist nicht nur unschädlich, sie stellt einen wichtigen Schutz zur Abwehr opportunistischer oder pathogener Keime dar.

Ein wesentliches Problem der Mundhygiene ist schlechter Atem, auch als Mundgeruch, Foetor ex oris oder Halitosis bekannt. Dieser Geruch wird durch die Zersetzung von Speiseresten und abgestorbenen Zellen der Schleimhaut durch Mikroorganismen gebildet. Der Befall mit grampositiven und gramnegativen Bakterien, Mycobionten und/oder Protozoen verursacht den Mundgeruch. Als Verursacher werden in der Literatur vor allem anaerobe gramnegative Bakterien genannt (z.B. Bad Breath - A multidisciplinary Approach. Eds: D. van Steenberghe, M. Rosenberg, Leuven University Press, Leuven 1996; 111-121).

Da durch Mundgeruch häufig soziale Kontakte beeinträchtigt werden, besteht ein großes Interesse der Betroffenen dem abzuhelfen oder vorzubeugen.

Gramnegative Keime kommen beispielsweise aus den Gattungen Bacteroides, Fusobacterium, Haemophilus, Neisseria, Porphyromonas, Prevotella, Treponema und Veillonella.

Grampositive Bakterien sind beispielsweise Vertreter der Gattungen Actinomyces, Eubacterium, Lactobacillus, Staphylococcus, Stomatococcus und Streptococcus.

Zu Vertretern der Mycobionten zählen beispielsweise Hefen (Protoascomycetes), und Schimmelpilze (Plectomycetes).

Zu den pathogenen und fakultativ pathogenen Keimen gehören beispielsweise aus der Gruppe der Hefen Candida-Arten (z.B. Candida albicans).

Die Aufgabe der vorliegenden Erfindung bestand darin, wirksame Verbindungen und Mittel gegen Mundgeruch bzw. gegen die an dessen Entstehung beteiligten Mikroorganismen bereitzustellen.

Die Erfindung betrifft primär die Verwendung einer Verbindung der Formel 1 oder einer Mischung aus zwei oder mehreren unterschiedlichen Verbindungen der Formel 1 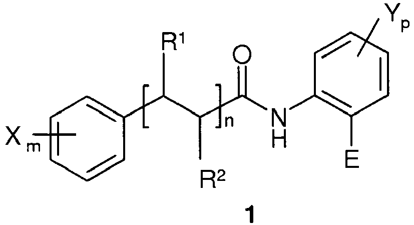 wobei für die Verbindung der Formel 1 oder jede Verbindung der Formel 1 in der Mischung gilt:
m = 0, 1, 2 oder 3,
p = 0, 1 oder 2,
n = 0, 1 oder 2,
wobei bei n = 1 oder 2 jeweils paarweise R¹ und R² jeweils H oder zusammen eine weitere chemische Bindung bedeuten,
wobei bei m = 1, 2 oder 3 jedes X, unabhängig von den anderen, OH, OAlkyl oder OAcyl bedeutet,
wobei bei p = 1 oder 2 jedes Y, unabhängig von den anderen, OH, OAlkyl oder OAcyl bedeutet,
wobei E = H oder einen Rest -COOR³ bedeutet,
R³ = H oder Alkyl, wobei R³ = H auch für die korrespondierenden pharmazeutisch akzeptablen Salze und Solvate steht,
zur Herstellung eines antibakteriell wirkenden Mittels, wobei das Mittel ein Mittel (i) zur Hemmung und/oder Verhinderung des Wachstums und/oder zur Abtötung von Mundgeruch verursachenden Organismen und/oder (ii) zur Bekämpfung oder Vermeidung von Mundgeruch ist.

Eine Verbindung der Formel 1 kann dabei in Form eines beliebigen Isomers oder Isomerengemisches vorliegen, also z.B. für n = 1 und R¹, R² = weitere chemische Bindung, als cis- oder trans-Isomer.

Für X oder Y = OAcyl gilt vorzugsweise: Acyl = CO-R mit R = -CH₃, linear oder verzweigter Alkylrest mit 2-30 C-Atomen.

Die vorteilhaften Ausgestaltungen der oder eine der Verbindungen der Formel 1 sind in den Unteransprüchen dargestellt.

Bevorzugt sind demnach Verbindungen der Formel 1, für die gilt:
n = 1 oder 2 und die Summe p + m > 0
und/oder p + m > 0 und mindestens einmal ist X oder Y aus der Gruppe ausgewählt, die aus OH und OAcyl besteht,
weiter bevorzugt Verbindungen der Formel 1, für die gilt:
n=1,
p+m≥ 2,
mit der Maßgabe, dass X und Y zusammen zumindest zweimal aus der Gruppe gewählt sind, die aus OH und OAcyl besteht.

Bevorzugt sind weiterhin Verbindungen der Formel 1, für die gilt:
n=1,
m = 1, 2 oder 3,
mit der Maßgabe, dass X zumindest einmal aus der Gruppe gewählt ist, die aus OH oder OAcyl besteht
und/oder
p = 1 oder 2,
mit der Maßgabe, dass Y zumindest einmal ausgewählt aus der Gruppe ist, die aus OH und OAcyl besteht.

Bevorzugt sind außerdem Verbindungen der Formel 1, für die gilt:
n=1
und
R¹ und R² bedeuten jeweils H oder zusammen eine weitere chemische Bindung.

Für weitere bevorzugte Ausgestaltungen der Verbindungen der Formel 1 gilt:
n = 0,
weiter bevorzugt:
n = 0, und
m + p > 2, mit der Maßgabe, dass zumindest zwei der Substituenten X und Y aus der Gruppe ausgewählt sind, die aus OH und OAcyl besteht.

Bevorzugt sind ebenfalls Verbindungen der Formel 1, für die gilt:
n=0,
n = 1,
p = 0,
X = OH und
E = H.

Ebenfalls sind bevorzugt Verbindungen der Formel 1, für die gilt:
R³ = CH₃ oder lineares oder verzweigtes Alkyl mit 2 bis 30 C-Atomen.

Hierin beschrieben wird ein Verfahren zum Hemmen und/oder Verhindern des Wachstums und/oder zum Abtöten von Mundgeruch verursachenden Mikroorganismen, mit folgendem Schritt:
- Kontaktieren Mundgeruch verursachender Mikroorganismen mit einer gegenüber diesen Mikroorganismen antimikrobiell wirksamen Menge einer Verbindung der Formel 1 oder einer Mischung aus zwei oder mehreren Verbindungen der Formel 1 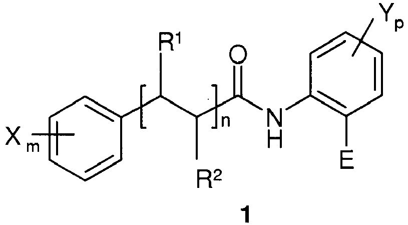 wobei für die Verbindung der Formel 1 oder jede Verbindung der Formel 1 in der Mischung gilt:
   m = 0, 1, 2 oder 3,
   p = 0, 1 oder 2,
   n = 0, 1 oder 2,
   wobei bei n = 1 oder 2 jeweils paarweise R¹ und R² jeweils H oder zusammen eine weitere chemische Bindung bedeuten,
   wobei bei m = 1, 2 oder 3 jedes X, unabhängig von den anderen, OH, OAlkyl oder OAcyl bedeutet,
   wobei bei p = 1 oder 2 jedes Y, unabhängig von den anderen, OH, OAlkyl oder OAcyl bedeutet,
   wobei E = H oder ein Rest -COOR³ bedeutet,
   R³ = H oder Alkyl, wobei R³ = H auch für die korrespondierenden pharmazeutisch akzeptablen Salze und Solvate steht.

Auch hier können die vorteilhaften Ausgestaltungen der oder einer der Verbindungen der Formel 1, wie oben dargestellt, eingesetzt werden.

Außerdem wird hierin beschrieben ein Verfahren zum Bekämpfen und/oder Vorbeugen von Mundgeruch, mit folgendem Schritt:
- Einbringen einer gegenüber Mundgeruch verursachenden Mikroorganismen antimikrobiell wirksamen Menge einer Verbindung der Formel 1 oder einer Mischung umfassend zwei oder mehrere Verbindungen der Formel 1 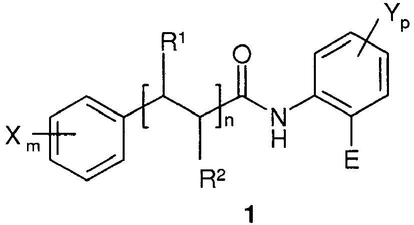 in die Mundhöhle und/oder den Rachenraum, wobei für die Verbindung der Formel 1 oder jede Verbindung der Formel 1 in der Mischung gilt:
   m = 0, 1, 2, oder 3,
   p = 0, 1 oder 2,
   n = 0, 1 oder 2, wobei bei n = 0 oder 2 jeweils paarweise R¹ und R² jeweils H oder zusammen eine weitere chemische Bindung bedeuten,
   wobei bei m = 1, 2 oder 3 jedes X, unabhängig von den anderen, OH, OAlkyl oder OAcyl bedeutet,
   wobei bei p = 1 oder 2 jedes Y, unabhängig von den anderen, OH, OAlkyl oder OAcyl bedeutet,
   wobei E = H oder einen Rest -COOR³ bedeutet,
   R³ = H oder Alkyl, wobei R³ = H auch für die korrespondierenden pharmazeutisch akzeptablen Salze und Solvate steht.

Auch hier können die vorteilhaften Ausgestaltungen der oder einer der Verbindungen der Formel 1, wie oben dargestellt, eingesetzt werden.

Die Erfindung betrifft auch bestimmte Produkte, die dazu bestimmt sind in die menschliche Mundhöhle eingebracht zu werden, dort eine bestimmte Zeit zu verbleiben und anschließend entweder geschluckt, d.h. verzehrt (z.B. Lebensmittel), oder wieder aus der Mundhöhle entfernt zu werden (z.B. Kaugummis), wobei das Produkt eine Verbindung der Formel 1 oder eine Mischung aus zwei oder mehreren Verbindungen der Formel 1 in einer zur Bekämpfung und/oder Vermeidung von Mundgeruch ausreichenden Menge umfasst. Hierzu zählen auch alle Stoffe oder Erzeugnisse, die dazu bestimmt sind, in verabeitetem, teilweise verabeitetem oder unverarbeitetem Zustand vom Menschen aufgenommen zu werden.

Auch hier können die vorteilhaften Ausgestaltungen der oder einer der Verbindungen der Formel 1, wie oben dargestellt, erfindungsgemäß eingesetzt werden.

Ein weiterer Gegenstand der vorliegenden Erfindung sind bestimmte Mundhygieneprodukte (mundhygienische Zubereitungen) umfassend oder bestehend aus einer oder mehreren erfindungsgemäß einzusetzenden Verbindungen der Formel 1 in einer zur Bekämpfung und/oder Vermeidung von Mundgeruch ausreichenden Menge.

Auch hier können die vorteilhaften Ausgestaltungen der oder einer der Verbindungen der Formel 1, wie oben dargestellt, erfindungsgemäß eingesetzt werden.

Unter Mundhygieneprodukten werden in der vorliegenden Erfindung die dem Fachmann geläufigen Formulierungen zur Reinigung und Pflege der Mundhöhle und des Rachenraumes sowie zur Erfrischung des Atems verstanden. Erfindungsgemäße Mundhygieneprodukte werden ausgewählt aus der Gruppe bestehend aus Zahncremes, Zahnpasten, Zahngele, Lutschpastillen, Lutschtabletten, Bonbons, Kaugummis, Kaubonbons und zahnpflegende Kaugummis.

Ein weiterer Gegenstand der vorliegenden Erfindung betrifft Mundwasser umfassend eine erfindungsgemäße Mischung umfassend oder bestehend aus:
(a) einer oder mehrerer Verbindungen der Formel 1 A, 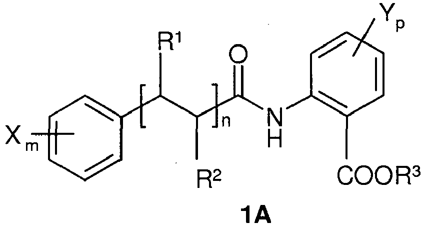 wobei für die Verbindung bzw. jede Verbindung der Formel 1A gilt:
   m = 0, 1, 2 oder 3,
   p = 0, 1 oder 2,
   n = 0, 1 oder 2,
   wobei bei n = 1 oder 2 jeweils paarweise R¹ und R² jeweils H oder zusammen eine weitere chemische Bindung bedeuten;
   wobei bei m = 1, 2 oder 3 jedes X, unabhängig von den anderen, OH, OAlkyl oder OAcyl bedeutet,
   wobei bei p = 1 oder 2 jedes Y, unabhängig von den anderen, OH, OAlkyl oder OAcyl bedeutet,
   R³ = H oder Alkyl, wobei R³ = H auch für die korrespondierenden kosmetisch oder pharmazeutisch akzeptablen Salze und Solvate steht,
   und
(b) einem oder mehreren Kühlwirkstoffen,
wobei die oder eine der Verbindungen der Formel 1 A in einer zur Bekämpfung und/oder Vermeidung von Mundgeruch ausreichenden Menge enthalten ist und der pH-Wert in einem Bereich von 6,5 - 8,0 liegt.

Die Verbindungen der Formel 1 A und insbesondere auch die Mischungen enthaltend (a) eine oder mehrere Verbindungen der Formel 1A und (b) einen oder mehrere Kühlwirkstoffe weisen zusätzlich eine juckreizlindernde und/oder Hautrötungen reduzierende Wirkung auf, wobei durch die Mischung von (a) und (b) eine synergistische Verstärkung dieser Wirkung hervorgerufen wird, so dass bereits geringe Einsatzkonzentrationen von Verbindung(en) der Formel 1A und der weiteren Verbindung ausreichen, um einen guten juckreizlindernden bzw. Rötungen reduzierenden Effekt zu bewirken.

Zur Verwendung der Verbindung(en) der Formel 1A in der erfindungsgemäßen Mischung besonders bevorzugt sind Verbindungen der Formel 1A, für die gilt:
n = 1 oder 2 und die Summe p + m > 0
und/oder
p + m > 0 und mindestens einmal ist X oder Y aus der Gruppe gewählt, die aus OH und OAcyl besteht.

Besonders bevorzugt ist der Einsatz einer Verbindung der Formel 1 A, für die gilt:
n=1,
p + m ≥ 2
mit der Maßgabe, dass X und Y zusammen zumindest zweimal aus der Gruppe gewählt sind, die aus OH und OAcyl besteht.

Ebenfalls bevorzugt ist die Verwendung einer Verbindung der Formel 1 A, für die gilt:
n=1,
wobei zudem gilt:
m = 1, 2 oder 3,
mit der Maßgabe, dass X zumindest einmal aus der Gruppe gewählt ist, die aus OH oder OAcyl besteht
   und/oder
p = 1 oder 2,
mit der Maßgabe, dass Y zumindest einmal aus der Gruppe gewählt ist, die aus OH und OAcyl besteht.

Falls n den Wert 1 besitzt, bedeuten R¹ und R² vorzugsweise jeweils H, R¹ und R² können jedoch auch zusammen eine weitere chemische Bindung bedeuten.

Die vorstehenden Erläuterungen bezogen sich im wesentlichen auf Verbindungen der Formel 1 mit n = 1.

Aber auch der Einsatz von Verbindungen der Formel 1 mit n = 0 ist vielfach bevorzugt.

Vorzugsweise gilt dann:
m + p ≥ 2,
mit der Maßgabe, dass zumindest zwei der Substituenten X und Y aus der Gruppe ausgewählt sind, die aus OH und OAcyl besteht.

In den als besonders bevorzugt dargestellten, durch ihr Formelbild angegebenen Verbindungen gilt jeweils R³ = H.

Anstelle dieser bevorzugten Verbindungen können jeweils auch die entsprechenden Verbindungen bevorzugt eingesetzt werden, für die gilt: R³ = CH₃ oder lineares oder verzweigtes Alkyl mit 2 bis 30 C-Atomen.

Einzelne zur Verwendung im Rahmen der vorliegenden Erfindung bevorzugte Kühlwirkstoffe sind nachfolgend aufgelistet. Der Fachmann kann die nachfolgende Liste um eine Vielzahl weiterer Kühlwirkstoffe ergänzen; die aufgelisteten Kühlwirkstoffe können auch in Kombination miteinander eingesetzt werden: I-Menthol, d-Menthol, racemisches Menthol, Menthonglycerinacetal (Handelsname: Frescolat^{®}MGA), Menthyllactat Handelsname: Frescolat^{®}ML, vorzugsweise handelt es sich bei Menthyllactat um I-Menthyllactat, insbesondere I-Menthyl-I-lactat), substituierte Menthyl-3-carbonsäureamide (z.B. Menthyl-3-carbonsäure-N-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, N-Acetylglycinmenthylester, Isopulegol, Menthylhydroxycarbonsäureester (z.B. Menthyl-3-hydroxybutyrat), Monomenthylsuccinat, 2-Mercaptocyclodecanon, Menthyl-2-pyrrolidin-5-oncarboxylat, 2,3-Dihydroxy-p-menthan, 3,3,5-trimethylcyclohexanonglycerinketal, 3-Menthyl-3,6-di- und trioxaalkanoate, 3-Menthyl methoxyacetat, Icilin.

Aufgrund ihres besonderen synergistischen Effekts bevorzugte Kühlwirkstoffe sind: I-Menthol, d-Menthol, racemisches Menthol, Menthonglycerinacetal (Handelsname: Frescolat^{®}MGA), Menthyllactat (vorzugsweise I-Menthyllactat, insbesondere I-Menthyl-I-lactat, Handelsname: Frescolat^{®}ML), substituierte Menthyl-3-carbonsäureamide (z.B. Menthyl-3-carbonsäure-N-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, Isopulegol.

Besonders bevorzugte Kühlwirkstoffe sind: I-Menthol, racemisches Menthol, Menthonglycerinacetal (Handelsname: Frescolat^{®}MGA), Menthyllactat (vorzugsweise I-Menthyllactat, insbesondere I-Menthyl-I-lactat, Handelsname: Frescolat^{®}ML), 3-Menthoxypropan-1,2-diol, 2-Hydroxyethylmenthylcarbonat, 2-Hydroxypropylmenthylcarbonat.

Ganz besonders bevorzugte Kühlwirkstoffe sind: I-Menthol, Menthonglycerinacetal (Handelsname: Frescolat^{®}MGA), Menthyllactat (vorzugsweise I-Menthyllactat, insbesondere I-Menthyl-I-lactat, Handelsname: Frescolat^{®}ML).

Die Einsatzkonzentration der erfindungsgemäß einzusetzenden Verbindungen der Formel 1A zur Juckreizlinderung kann zwar - je nach Substanz - im Konzentrationsbereich von 0,0001 bis zu 10 Gewichtsprozent liegen, wie ja auch schon gemäß der WO 2004/047833. Bevorzugt ist jedoch der Einsatz einer niedrigen Konzentration an der oder den Verbindungen der Formel 1 A. Bevorzugt ist insbesondere ein Konzentrationsbereich von 0,001 bis 1 Gewichtsprozent und besonders bevorzugt ist ein Bereich von 0,01 bis 0,2 Gewichtsprozent, jeweils bezogen auf die Gesamtmasse ein anwendungsbereiten kosmetischen oder pharmazeutischen Endprodukts.

Die Einsatzkonzentration der erfindungsgemäß einzusetzenden Kühlwirkstoffe zur Juckreizlinderung liegt je nach Substanz vorzugsweise im Konzentrationsbereich von 0,01 bis 20 Gewichtsprozent und bevorzugt im Konzentrationsbereich von 0,1 bis 5 Gewichtsprozent, bezogen auf die Gesamtmasse eines anwendungsbereiten kosmetischen oder pharmazeutischen Endprodukts.

Besonders bevorzugt sind erfindungsgemäße Mundwässer, in denen das Gewichtsverhältnis der Gesamtmenge von Verbindungen der Formel 1A zur Gesamtmenge an Kühlwirkstoffen im Bereich von 1:100 bis 1:2 liegt, bevorzugt im Bereich von 1:50 bis 1:5 und besonders bevorzugt im Bereich von 1:30 bis 1:10. Der Gewichtsanteil der Kühlwirkstoffe überwiegt also vorzugsweise im Vergleich mit dem Gewichtsanteil der Verbindungen der Formel 1A.

Die erfindungsgemäßen Mundwässer lassen sich mit einer Vielzahl weiterer Bestandteile kombinieren, wodurch sich bevorzugte kosmetische und/oder pharmazeutische Mischungen bzw. Produkte ergeben.

Die erfindungsgemäß einzusetzenden Verbindungen der Formel 1 oder der Formel 1 A sind Verbindungen, die weitgehend universell in die verschiedensten Darreichungsformen an Mundhygieneprodukten eingearbeitet werden können, ohne auf eine oder wenige spezielle Darreichungsformen festgelegt zu sein, d.h. dass die erfindungsgemäß einzusetzenden Verbindungen der Formel 1 oder der Formel 1A mit einer großen Vielzahl an üblichen kosmetischen Hilfs- und Zusatzstoffen harmonieren. Verbindungen der Formel 1 und/oder 1 A können im Bedarfsfalle in hoher Konzentration in aprotischen, dipolaren Lösungsmitteln wie z. B. Dimethylsulfoxid, Dimethylformamid, aber auch in anderen Lösungsmitteln oder Lösungsmittelkombinationen (vor)gelöst werden.

Es wurde gefunden, dass bei Verwendung der erfindungsgemäß einzusetzenden Verbindungen der Formel 1 oder Formel 1 A das Wachstum von Mikroorganismen der Mundhöhle, insbesondere von grampositiven und gramnegativen Bakterien, verhindert oder unterdrückt werden kann.

Es hat sich ferner herausgestellt, dass die erfindungsgemäß einzusetzenden Verbindungen der Formel 1 oder Formel 1A die Bildung von schlechtem Atem wirkungsvoll verringern oder beseitigen können beziehungsweise dessen Entstehung vorbeugen.

Unter Verwendung der erfindungsgemäß einzusetzenden Verbindungen der Formel 1 oder Formel 1 A und Mundhygieneprodukten, umfassend oder bestehend aus einer oder mehrerer Verbindungen der Formel 1 oder Formel 1 A ist eine wirksamen Bekämpfung von schlechtem Atem möglich ohne dabei die physiologische Flora des Mund- und Rachenraums nennenswert zu schädigen.

Der Stand der Technik lieferte keinen Hinweis auf die erfindungsgemäße Verwendung von Verbindungen der Formel 1 oder Formel 1 A als Mittel zur Verminderung, Beseitigung bzw. Vorbeugung zur Entstehung von Mundgeruch, sowie bakterielle Phänomene wie z.B. Karies, Parodontitis, Plaque, Gingivitis.

Bevorzugte erfindungsgemäß einzusetzende Verbindungen der Formel 1 oder Formel 1 A sind:

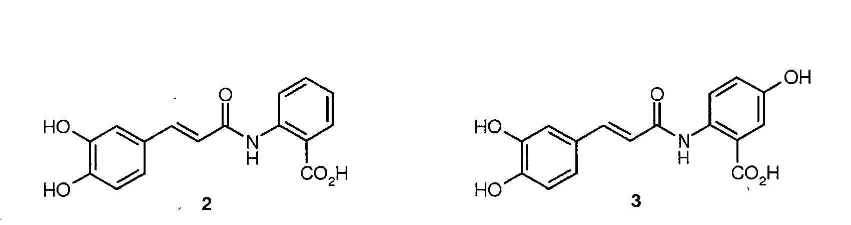

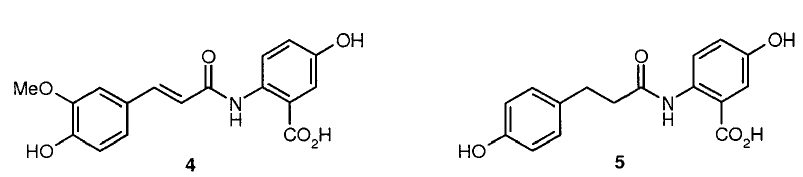

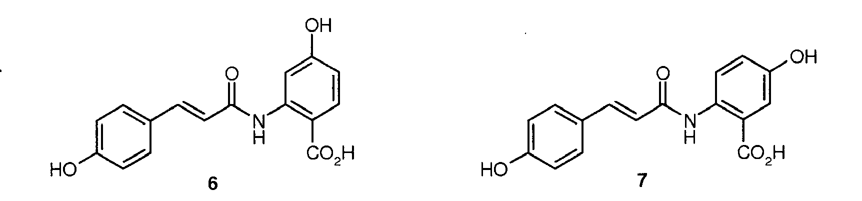

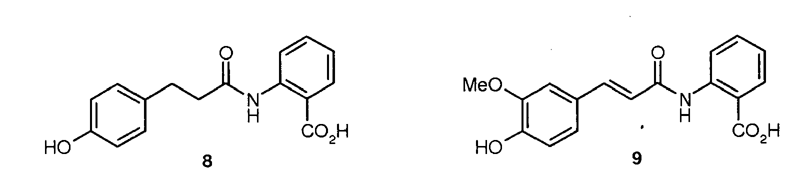

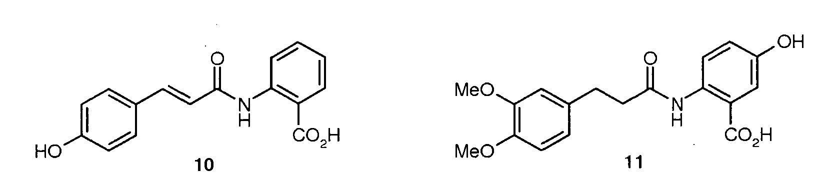

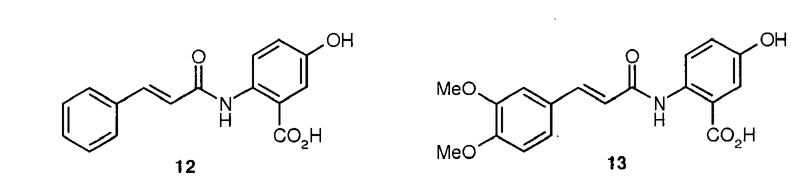

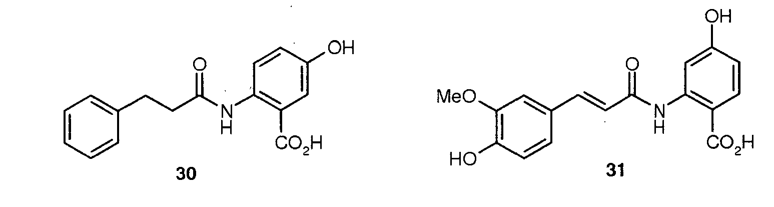

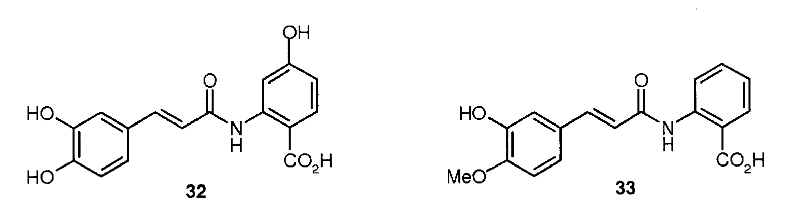

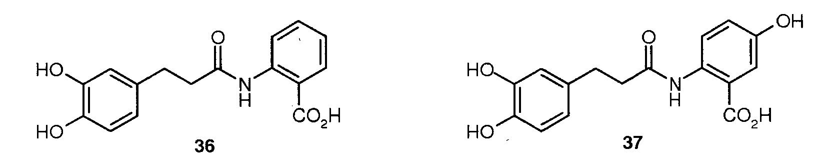

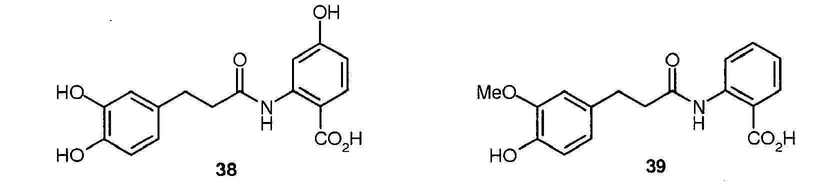

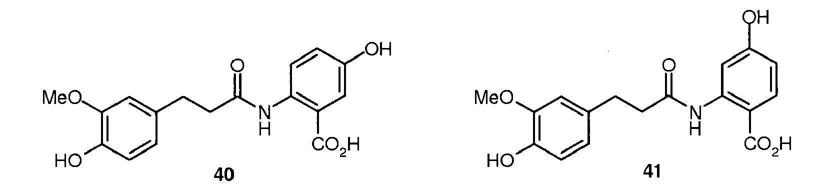

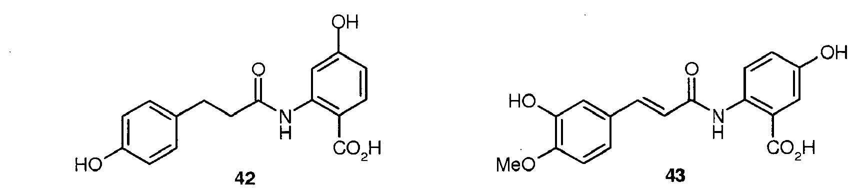

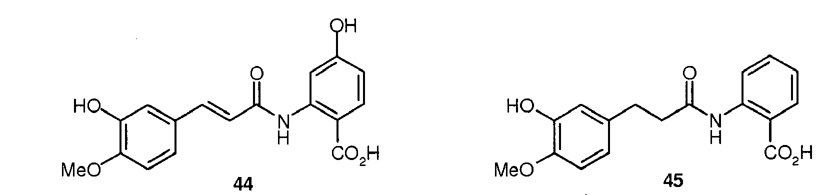

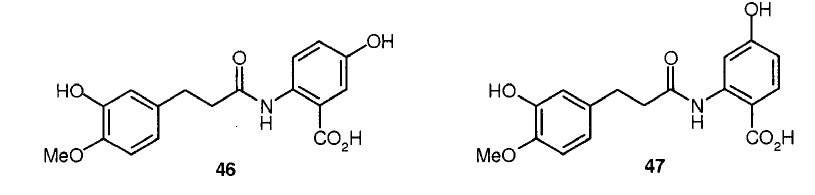

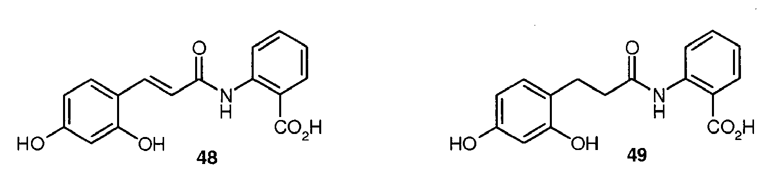

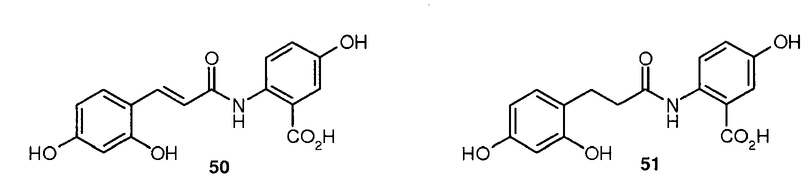

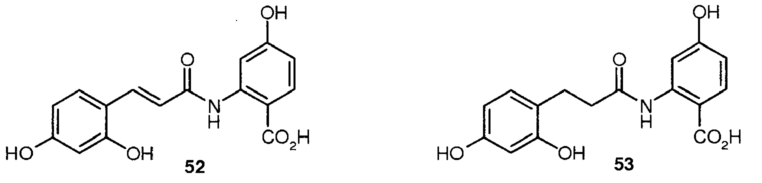

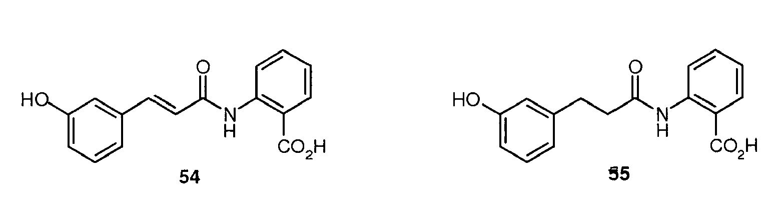

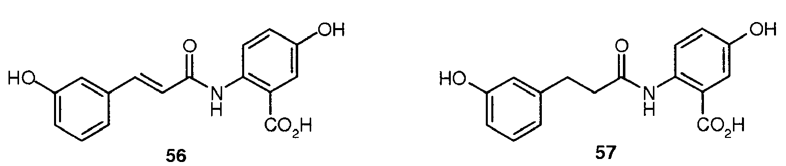

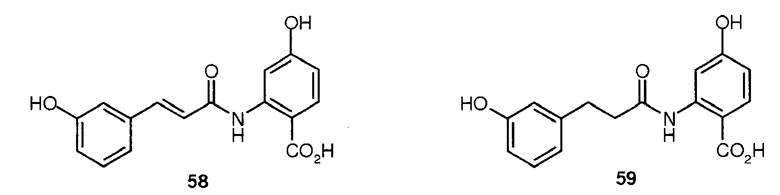

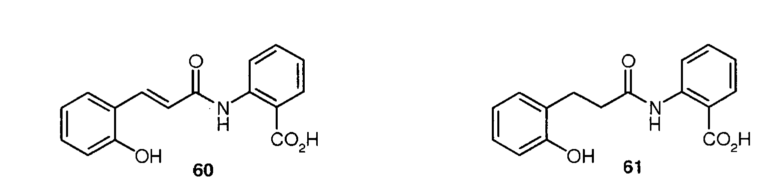

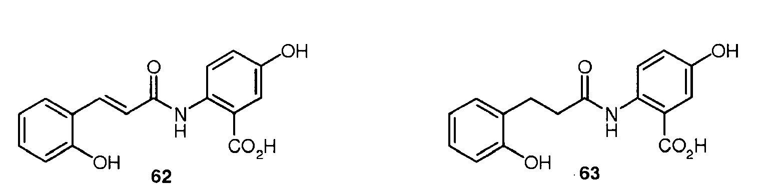

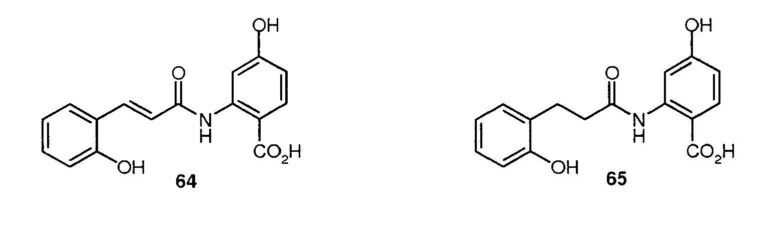

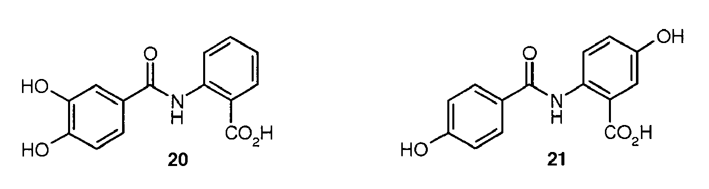

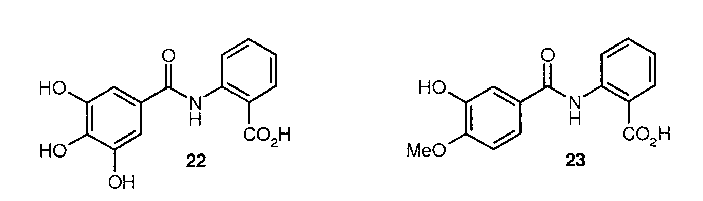

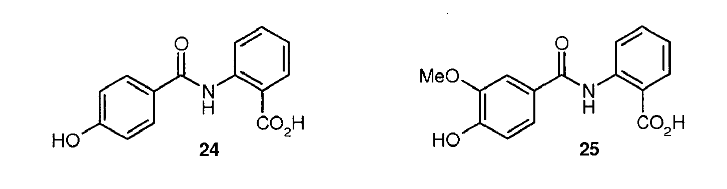

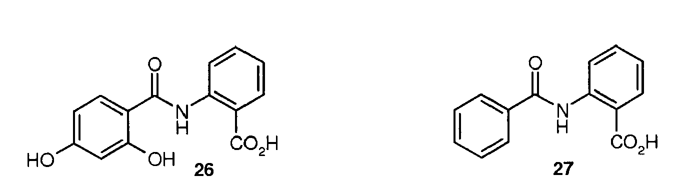

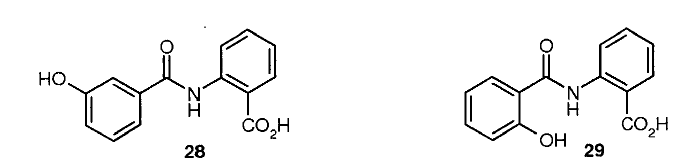

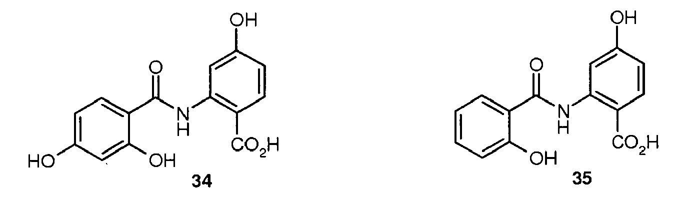

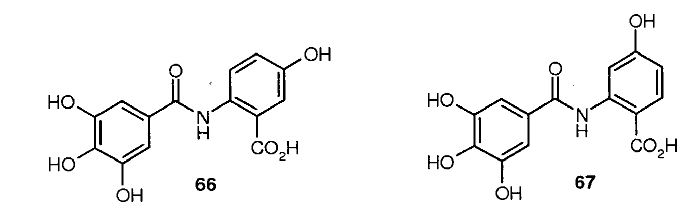

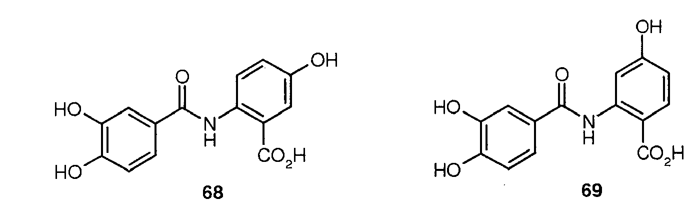

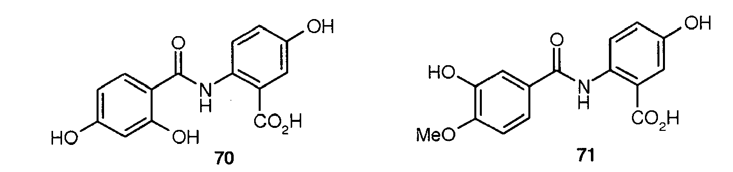

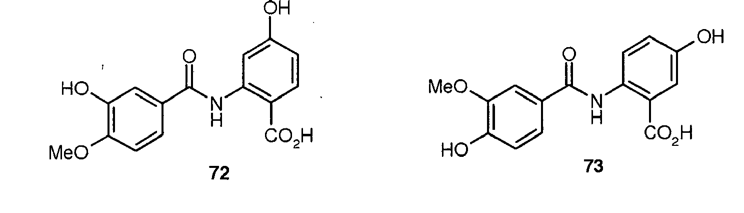

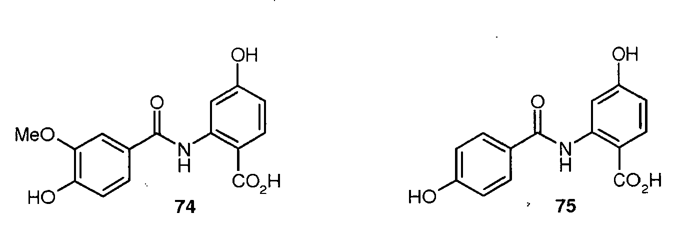

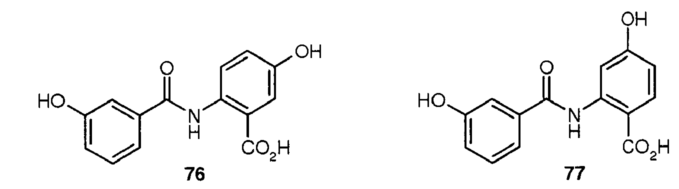

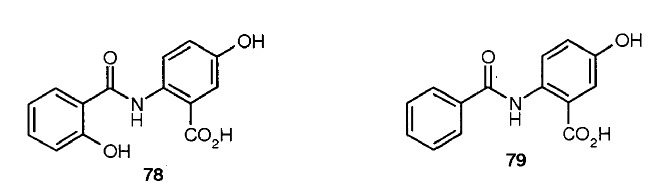

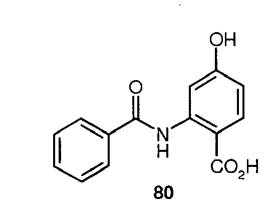

Bevorzugte erfindungsgemäß einzusetzende Verbindungen der Formel 1 sind:

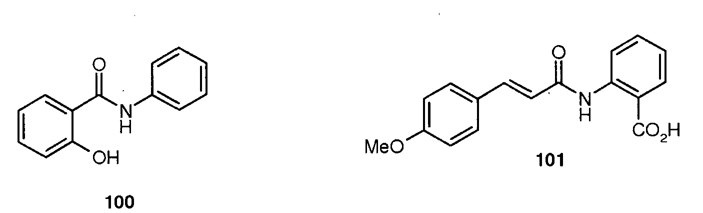

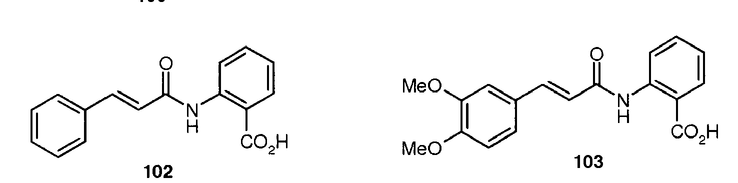

Besonders bevorzugte erfindungsgemäß einzusetzende Verbindungen der Formel 1 auf Grund ihrer sehr guten Wirkung gegen anaerobe gramnegative Bakterien sind:

Verbindungen der Formeln 28, 102, 24, 25, 10, 12, 101, 27, 29, 26, 23, 3, 100, 7, 9, 2, 8, 20, 4, 13, 75, 102 und 103.

Am meisten bevorzugt sind dabei wiederum die gegen Mundgeruch besonders wirksamen (siehe insoweit auch die Ergebnisse des in-vitro Tests zur Mundgeruchsreduktion) Verbindungen der Formeln 20, 8, 2, 9, 7, 100, 23, 26, 29, 27, 101, 12, 10, 25, 24, 102, 28 und 3:

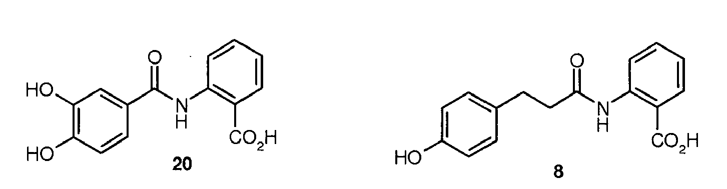

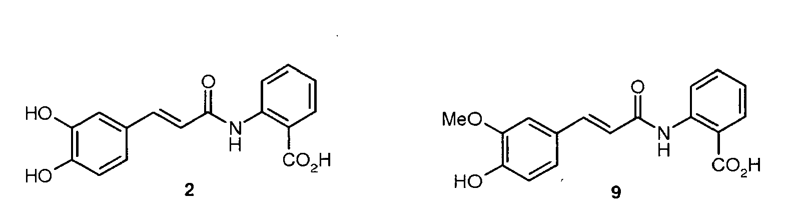

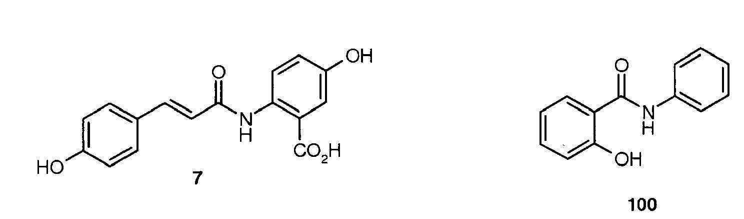

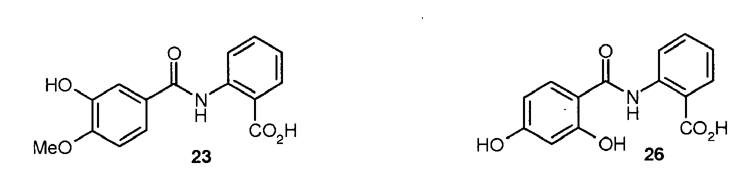

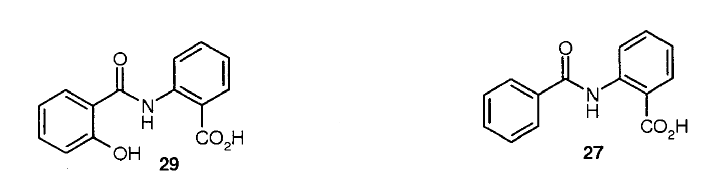

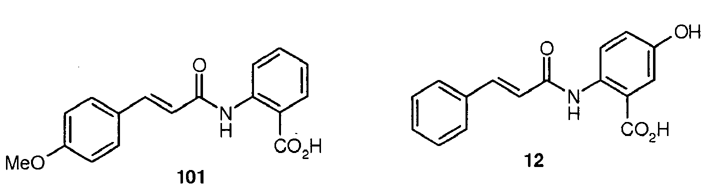

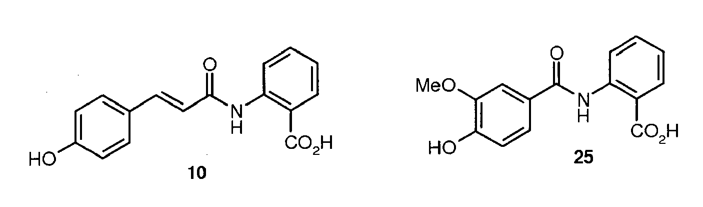

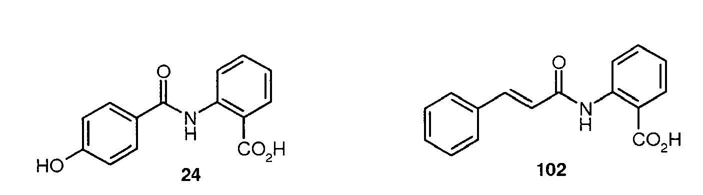

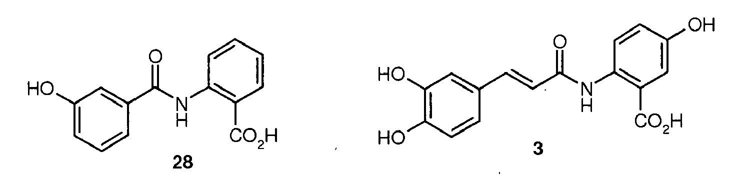

Besonders bevorzugte erfindungsgemäß einzusetzende Verbindungen der Formel 1A aufgrund ihrer sehr guten Wirkung gegen anaerobe gramnegative Bakterien sind: 28, 24, 25, 10, 12, 27, 29, 26, 23, 3, 7, 9, 2, 8, 20, 4, 13 und 75.

Am meisten bevorzugt sind dabei wiederum die gegen Mundgeruch besonders wirksamen (siehe insoweit auch die Ergebnisse des in-vitro Tests zur Mundgeruchsreduktion) Verbindungen der Formeln: 20, 8, 2, 9, 7, 23, 26, 29, 27, 12, 10, 25, 24, 28 und 3.

Es ist ebenfalls vorteilhaft, natürliche oder synthetische Stoffe oder Stoffgemische einzusetzen, welche sich durch einen wirksamen Gehalt an den erfindungsgemäßen Verbindungen der Formel 1 oder der Formel 1A auszeichnen, wie Extrakte der Gattungen Avena, Dianthus, Silene oder Melandrium. Die erfindungsgemäßen Verbindungen können einzeln oder in Kombination mit anderen erfindungsgemäßen Verbindungen und weiteren Aromastoffen verwendet werden. Bevorzugt sind neben der jeweiligen Einzelverbindungen Kombinationen aus zwei oder drei erfindungsgemäßen Verbindungen und gegebenenfalls weiteren Aromastoffen.

Es wurde gefunden, dass die erfindungsgemäßen Verbindungen das Wachstum von grampositiven und gramnegativen Bakterien, Mycobionten und/oder Protozoen des Mund- und Rachenraums, bevorzugt von denjenigen Bakterien, Mycobionten und/oder Protozoen, die den Mundgeruch hervorrufen, reduzieren und/oder verhindern.

Es wurde zudem gefunden, dass die erfindungsgemäßen Verbindungen die Bildung von den Mundgeruch verursachenden Komponenten verhindern und/oder reduzieren.

Insbesondere sind die erfindungsgemäß verwendeten Verbindungen der Formel 1 oder Formel 1A befähigt, das Wachstum von Mundgeruch verursachende Keime, insbesondere der Arten Eubacterium, Fusobacterium, Haemophilus, Neisseria, Porphyromonas, Prevotella, Treponema und Veillonella, insbesondere Fusobacterium nucleatum, Porphyromonas endodontalis, Porphyromonas gingivalis, Prevotella intermedia, Prevotella loeschii, Treponema denticola und Veillonella parvula, zu reduzieren und/oder zu verhindern.

Ferner war erstaunlich, dass die erfindungsgemäßen Verbindungen der Formel 1 oder der Formel 1A besonders gut wirksam sind gegen den typischerweise am Morgen nach dem Aufstehen wahrzunehmenden, besonders ausgeprägten morgendlichen Mundgeruch.

Die erfindungsgemäßen Verbindungen der Formel 1 oder der Formel 1A werden vorzugsweise in Mundhygieneprodukten (mundhygienischen Zubereitungen) eingesetzt, dabei bevorzugt mit einem Gesamtgehalt im Bereich von 0,0005 - 5,0 Gew.-% (entsprechend 5 - 50000 ppm), besonders bevorzugt im Bereich von 0,001 - 2,0 Gew.-% (entsprechend 10 - 20000 ppm), insbesondere im Bereich von 0,0025 - 1,5 Gew.-% (entsprechend 25 - 15000 ppm), jeweils bezogen auf das Gesamtgewicht der Zubereitung. Weiter bevorzugt liegt der Gesamtgehalt an erfindungsgemäß einzusetzenden Verbindungen der Formel 1 oder der Formel 1A im Bereich von 0,005 - 1,0 Gew.-% (entsprechend 50 - 10000 ppm), besonders bevorzugt im Bereich von 0,01 - 0,5 Gew.-% (entsprechend 100 - 5000 ppm), jeweils bezogen auf das Gesamtgewicht der Zubereitung.

In eigenen Untersuchungen wurde ferner gefunden, dass die erfindungsgemäß einzusetzenden Verbindungen der Formel 1 oder der Formel 1A bzw. Mischungen aus zwei oder mehr unterschiedlichen Verbindungen der Formel 1 oder der Formel 1A einen entweder nur geringen oder (im Wesentlichen) neutralen Eigengeschmack aufweisen, insbesondere in den oben für Mundhygieneprodukte angegebenen Konzentrationen, wodurch sich die Verbindungen der Formel 1 oder der Formel 1A hervorragend in oral zu applizierende Produkte wie Mundhygieneprodukte einarbeiten lassen ohne diese dabei geschmacklich (nennenswert) zu verändern.

Es ist vorteilhaft die erfindungsgemäßen mundhygienischen Zubereitungen abzupuffern. Vorteilhaft ist ein pH-Bereich von 3,5 - 10,0. Besonders günstig ist es, den pH-Wert in einem Bereich von 6,5 - 8,0 zu wählen.

Die erfindungsgemäßen Verbindungen der Formel 1 oder der Formel 1A lassen sich problemlos in gängige mundhygienische Formulierungen für Mundhygieneprodukte einarbeiten. Bevorzugte Mundhygieneprodukte sind Zahncremes, Zahnpasten, Zahngele, Mundwässer, Mundspülungen, Flüssigkeiten zum Gurgeln, Mund- oder Rachensprays (Pump- oder Aerosolspray), Lutschpastillen, Lutschtabletten, Bonbons, Kaugummis, Kaubonbons und zahnpflegende Kaugummis.

Die erfindungsgemäßen mundhygienischen Zubereitungen können Hilfsstoffe (Zusatzstoffe) enthalten, wie sie üblicherweise in solchen Zubereitungen verwendet werden, insbesondere einen oder mehrere Stoffe aus folgender Gruppe:

Konservierungsmittel, Abrasiva (schleifende Mittel), weitere antibakterielle Mittel, entzündungshemmende Mittel, irritationsverhindernde Mittel, irritationshemmende Mittel, weitere antimikrobielle Mittel, Antioxidantien, Adstringentien, Antistatika, Binder, (mineralische) Füllstoffe, Puffer, Trägermaterialien, Chelatoren (Chelatbildner), reinigende Mittel, pflegende Mittel, oberflächenaktive Substanzen, deodorierende Mittel, Emulgatoren, Enzyme, Fasern, Filmbildner (filmbildende Substanzen), Fixateure, Schaumbildner, Substanzen zum Verhindern des Schäumens, Schaumstabilisatoren, Schaumbooster, gelierende Mittel, gelbildende Mittel, feuchtigkeitsspendende Mittel (Moisturizer), anfeuchtende Substanzen, feuchthaltende Substanzen, bleichende Mittel, aufhellende Mittel (z.B. Wasserstoffperoxid), imprägnierende Mittel, reibungsverringernde Mittel, Gleitmittel, geruchs- und/oder geschmacksmodulierende Mittel, geruchs- un d/oder geschmacksreduzierende Mittel, geruchs- und/oder geschmacksverstärkende Mittel, Trübungsmittel, plastifizierende Mittel, deckfähige Mittel, Glanzmittel, Silikone, (schleim)hautkühlende Mittel (Kühlwirkstoffe), (schleim)hautberuhigende Mittel, (schleim)hautreinigende Mittel, (schleim)hautpflegende Mittel, (schleim)hautheilende Mittel, schleimhautschützende Mittel, UV-Filter, Stabilisatoren, suspendierende Mittel, Vitamine, fette Öle, Wachse, Fette, Phospholipide, gesättigte Fettsäuren, ein- oder mehrfach ungesättigte Fettsäuren, alpha-Hydroxysäuren, Polyhydroxysäuren, Verflüssiger, Farbstoffe, farbschützende Mittel, Pigmente, Tenside, Elektrolyte, Silikonderivate, Polyole, organische Lösungsmittel, Kieselsäuren, Calciumcarbonat, Calciumhydrogenphosphat, Aluminiumoxid, Fluoride, Zink-, Zinn-, Kalium-, Natrium- und Strontiumsalze, Pyrophosphate, Hydroxyapatite.

Sofern die mundhygienische Zubereitung eine Lösung oder Lotion darstellt, können beispielsweise als Lösungsmittel verwendet werden: Wasser oder wäßrige Lösungen, Öle, wie Triglyceride der Caprin- oder der Caprylsäure oder auch Alkohole, Diole oder Polyole niedriger C-Zahl sowie deren Ether, vorzugsweise Ethanol, Isopropanol, Propylenglykol, Glycerin, Ethylenglykol. Insbesondere werden Gemische der vorstehend genannten Lösungsmittel verwendet.

Geschmack- und Aromastoffe im Sinne der vorliegenden Erfindung sind sensorisch wirksame Substanzen, welche flüchtig (Aromastoffe) oder nichtflüchtig (Geschmackstoffe) sind. Die flüchtigen Aromastoffe können vom Menschen sowohl orthonasal als auch retronasal wahrgenommen werden. Die Geschmackstoffe interagieren mit den Geschmacksrezeptoren der Zunge und sind für die gustatorischen (geschmacklichen) Eindrücke süß, sauer, bitter, salzig und umami verantwortlich, daneben werden auch andere, oftmals trigeminale Reize, wie beispielsweise scharfe, brennende, kühlende, elektrisierende ("tingling") oder prickelnde Effekte, wahrgenommen.

Geschmackstoffe im Sinne der vorliegenden Erfindung umfassen somit unter anderem (schleimhaut)kühlende Mittel, (schleimhaut)wärmende Mittel, scharf schmeckene Stoffe, Süßstoffe, Zuckeraustauschstoffe, organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), und verzehrbare mineralische Salze (z.B. Natriumchlorid, Kaliumchlorid, Magnesiumchlorid, Natriumphosphate).

Vorteilhafte Aromastoffe, die als Bestandteil der erfindungsgemäßen Zubereitungen geeignet sind, finden sich z.B. in S. Arctander, Perfume and Flavor Chemicals, Vol. I und II, Montclair, N. J. 1969, Eigenverlag, oder K. Bauer, D. Garbe und H. Surburg, Common Fragrance and Flavor Materials, 4th Edition, Wiley-VCH, Weinheim 2001 und können beispielsweise gewählt sein aus folgenden Stoffklassen: aliphatische Ester (gesättigt und ungesättigt) z.B. Ethylbutyrat, Allylcapronat; aromatische Ester z.B. Benzylacetat, Methylsalicylat; cyclische Alkohole z.B. Menthol; aliphatische Alkohole z.B. Isoamylalkohol, 3-Octanol; aromatische Alkohole z.B. Benzylalkohol; aliphatische Aldehyde (gesättigt und ungesättigt) z.B. Acetaldehyd, Isobutyraldehyd; aromatische Aldehyde z.B. Benzaldehyd; Vanillin; Ketone z.B. Menthon, Carvon; cyclische Ether z.B. 4-Hydroxy-5-methylfuranon; aromatische Ether z.B. p-Methoxybenzaldehyd, Guajacol; Lactone z.B. gamma-Decalacton; Terpene z.B. Limonen, Linalool, Terpinen, Terpineol, Citral. Vorzugsweise wird eine Mischung von 2, 3, 4, 5, 6, 7, 8, 9, 10 oder mehr Aromastoffen eingesetzt, die wiederum vorzugsweise mindestens einen, bevorzugt 2, 3, 4, 5 oder mehr Aromastoffe aus den oben genannten Stoffklassen umfasst.

Optisch aktive Aromastoffe können dabei in enantiomerenreiner Form eingesetzt werden oder als beliebige Gemische der beiden Enantiomere. Gleiches gilt für (E)/(Z)-Isomere und Diastereomere.

Es ist von besonderem Vorteil, wenn die erfindungsgemäßen Zubereitungen mindestens einen Aromastoff, vorzugsweise 2, 3, 4, 5, 6, 7, 8, 9, 10 oder mehr Aromastoffe, ausgewählt aus folgender Gruppe enthalten: Menthol (vorzugsweise 1-Menthol und/oder racemisches Menthol), Anethol, Anisol, Anisaldehyd, Anisalkohol, (racemisches) Neomenthol, Eucalyptol (1,8-Cineol), Menthon (vorzugsweise L-Menthon), Isomenthon (vorzugsweise D-Isomenthon), Isopulegol, Menthylacetat (vorzugsweise L-Menthylacetat), Menthylpropionat, Carvon (vorzugsweise (-)-Carvon, gegebenenfalls als Bestandteil eines Krauseminzöls (Spearmintöls)), Methylsalicylat (gegebenenfalls als Bestandteil eines Wintergrünöls), Eugenolacetat, Isoeugenolmethylether, beta-Homocyclocitral, Eugenol, Isobutyraldehyd, 3-Octanol, Dimethylsulfid, Hexanol, Hexanal, trans-2-Hexenal, cis-3-Hexenol, 4-Terpineol, Piperiton, Linalool, 8-Ocimenylacetat, Isoamylalkohol, Isovaleraldehyd, alpha-Pinen, beta-Pinen, Limonen (vorzugsweise D-Limonen, gegebenenfalls als Bestandteil eines etherischen Öls), Piperiton, trans-Sabinenhydrat, Menthofuran, Caryophyllen, Germacren D, Zimtaldehyd, Mintlacton, Thymol, gamma-Octalacton, gamma-Nonalacton, gamma-Decalacton, (1,3E,5Z)-Undecatrien, 2-Butanon, Ethylformiat, 3-Octylacetat, Isoamylisovalerianat, cis- und trans-Carvylacetat, p-Cymol, Damascenon, Damascon, cis-Rosenoxid, trans-Rosenoxid, Fenchol, Acetaldehyddiethylacetal, 1, 1-Ethoxyethylacetat, cis-4-Heptenal, cis-Jasmon, Methyldihydrojasmonat, 2'-Hydroxypropiophenon, Menthylmethylether, Myrtenylacetat, 2-Phenylethylalkohol, 2-Phenylethylisobutyrat, 2-Phenylethylisovalerat, Geraniol, Nerol, Viridiflorol.

Bei chiralen Verbindungen können die (bevorzugten) Aromastoffe als Racemat oder als einzelnes Enantiomer oder als enantiomerenangereichertes Gemisch vorliegen.

Insbesondere wird eine erfrischende Wirkung im Mund-, Rachen- und/oder Nasenraum erzielt, wenn die erfindungsgemäßen Zubereitungen mindestens einen Aromastoff, vorzugsweise 2, 3, 4, 5 oder mehr Aromastoffe, aus folgender Gruppe enthalten: I-Menthol, racemisches Menthol, Anethol, Anisaldehyd, Anisalkohol, Neomenthol, Eucalyptol (1,8-Cineol), L-Menthon, D-Isomenthon, Isopulegol, L-Menthylacetat, (-)-Carvon, Methylsalicylat, trans-2-Hexenal, cis-3-Hexenol, 4-Terpineol, Linalool, 8-Ocimenylacetat, alpha-Pinen, D-Limonen, (+)-Menthofuran, Zimtaldehyd, Menthylmethylether.

Menthol kann dabei in reiner Form (natürlich oder synthetisch) und/oder als Bestandteil natürlicher Öle und/oder Menthol-haltiger Fraktionen natürlicher Öle eingesetzt werden, im Speziellen in Form etherischer (d.h. mittels Wasserdampfdestillation gewonnenen) Öle bestimmter Mentha-Spezies, insbesondere aus Mentha arvensis (Ackerminze, US-sprachlich auch cornmint genannt) und aus Mentha piperita (US-sprachlich peppermint genannt), diese schließen Mentha piperita Öle mit regionalen Herkunftsbezeichnungen spezieller Anbaugebiete wie Willamette, Yakima und Madras sowie Öle vom Typ der vorgenannten Bezeichnungen ein.

(-)-Carvon kann dabei in reiner Form (natürlich oder synthetisch) und/oder als Bestandteil natürlicher Öle und/oder Menthol-haltiger Fraktionen natürlicher Öle eingesetzt werden, im Speziellen in Form etherischer (d.h. mittels Wasserdampfdestillation gewonnenen) Öle bestimmter Mentha-Spezies, insbesondere aus Mentha cardiaca oder Mentha spicata.

Anethol kann dabei als cis- oder trans-Anethol oder in Form von Mischungen der Isomere verwendet werden. Anethol kann dabei in reiner Form (natürlich oder synthetisch) und/oder als Bestandteil natürlicher Öle und/oder Anethol-haltigen Fraktionen natürlicher Öle eingesetzt werden, insbesondere in Form von Anisöl, Sternanisöl oder Fenchelöl oder Anethol-haltige Fraktionen davon.

Eucalyptol kann in reiner Form (natürlich oder synthetisch) und/oder als Bestandteil natürlicher Öle und/oder Eucalyptol-haltigen Fraktionen natürlicher Öle eingesetzt werden, beispielsweise in Form von Lorbeer(blatt)öl, bevorzugt sind jedoch Eucalytusöle von Eucalyptus fruticetorum und/oder Eucalyptus globulus und/oder Eucalyptol-haltigen Fraktionen davon.

Besonders geeignete Substanzen mit kühlender und/oder erfrischender Wirkung im Mund-, Rachen- und/oder Nasenraum sind: Menthol, Menthon, Isomenthon, 1,8-Cineol (Eucalyptol), (-)-Carvon, 4-Terpineol, Thymol, Methylsalicylat, L-Menthylmethylether.

Besonders geeignete Aroma- und/oder Geschmackstoffe im Sinne der Erfindung sind ferner etherische Öle und Extrakten, Tinkturen und Balsame, wie beispielsweise Anisöl, Basilikumöl, Bergamotteöl, Bittermandelöl, Campheröl, Citronellöl, Citronenöl; Eucalyptus-citriodora-Öl, Eucalyptusöl, Fenchelöl, Grapefruitöl, Ingweröl, Kamillenöl, Krauseminzöl, Kümmelöl, Limetteöl, Mandarinenöl, Muskatöl (insbesondere Muskatblütenöl = Maces Öl, Mace Oil), Myrrhe Öl, Nelkenöl, Nelkenblütenöl, Orangenöl, Oreganoöl, Petersilien(samen)öl, Pfefferminzöl, Rosmarinöl, Salbeiöl (Muskateller Salbei, Dalmatisches oder Spanisches Sage Öl), Sternanisöl, Thymianöl, Vanilleextrakt, Wacholderöl (insbesondere Wacholderbeeröl), Wintergrünöl, Zimtblätteröl; Zimtrindenöl, sowie Fraktionen davon, bzw. daraus isolierte Inhaltsstoffe.

Bevorzugte Kühlwirkstoffe zur Verwendung im Rahmen der vorliegenden Erfindung zur Einarbeitung in die erfindungsgemäßen Zubereitungen sind nachfolgend aufgelistet. Der Fachmann kann die nachfolgende Liste um eine Vielzahl weiterer Kühlwirkstoffe ergänzen; die Kühlwirkstoffe können auch in Kombination miteinander eingesetzt werden. Vorzugsweise umfassen die erfindungsgemäßen Zubereitungen mindestens einen Kühlwirkstoff, vorzugsweise zwei mehr Kühlwirkstoffe, ausgewählt aus der Gruppe bestehend aus:

Menthonglycerinacetal (Handelsname: Frescolat^{®}MGA, Symrise GmbH & Co. KG, Deutschland), Menthyllactat (Handelsname: Frescolat^{®}ML, Symrise GmbH & Co. KG, Deutschland, vorzugsweise handelt es sich bei Menthyllactat um I-Menthyllactat, insbesondere 1-Menthyl-I-lactat), substituierte Menthyl-3-carbonsäureamide (z.B. Menthyl-3-carbonsäure-N-ethylamid, auch als WS-3 bekannt), 2-Isopropyl-N-2,3-trimethylbutanamid (auch als WS-23 bekannt), substituierte Cyclohexancarbonsäureamide, 3-Menthoxypropan-1,2-diol, 2-Hydroxyethylmenthylcarbonat, 2-Hydroxypropylmenthylcarbonat, N-Acetylglycinmenthylester, Isopulegol, Menthylhydroxycarbonsäureester (z.B. Menthyl-3-hydroxybutyrat), Monomenthylsuccinat, 2-Mercaptocyclodecanon, Menthyl-2-pyrrolidin-5-oncarboxylat, 2,3-Dihydroxy-p-menthan, 3,3,5-trimethylcyclohexanonglycerinketal, 3-Menthyl-3,6-di- und -trioxaalkanoate, 3-Menthyl methoxyacetat, Icilin.

Besonders bevorzugte Kühlwirkstoffe sind: Menthonglycerinacetal (Handelsname: Frescolat^{®}MGA), Menthyllactat (vorzugsweise I-Menthyllactat, insbesondere I-Menthyl-I-lactat, Handelsname: Frescolat^{®}ML), substituierte Menthyl-3-carbonsäureamide (z.B. Menthyl-3-carbonsäure-N-ethylamid), 2-Isopropyl-N-2,3-trimethylbutanamid, 3-Menthoxypropan-1,2-diol, 2-Hydroxyethylmenthylcarbonat, 2-Hydroxypropylmenthylcarbonat, Isopulegol und Monomenthylsuccinat.

Erfindungsgemäß bevorzugt sind erfindungsgemäße Zubereitungen, die 1-Menthol und mindestens eine, besonders bevorzugt mindestens zwei, Kühlsubstanzen enthalten.

Vorzugsweise enthält eine erfindungsgemäße Zubereitung eine Mischung von Geschmack- und/oder Aromastoffen, welche einer erfindungsgemäßen Zubereitung einen herbalen (krautigen), minzigen, zimtigen, nelkigen, Wintergrün und/oder fruchtigen Charakter verleiht.

Ferner ist es dabei von Vorteil, wenn eine erfindungsgemäße Zubereitung zusätzlich einen oder mehrere Kühlwirkstoffe umfasst, vorzugsweise aus der oben bevorzugt genannten Gruppe der Kühlwirkstoffe. Durch diese bevorzugten Kombinationen wird in besonderem Maße eine erfrischende Wirkung im Mund-, Rachen- und/oder Nasenraum erzielt.

Gemäß einer weiteren bevorzugten Ausführungsform werden die erfindungsgemäß einzusetzenden Geschmack- und/oder Aromastoffe vor deren Verwendung bei der Herstellung der erfindungsgemäßen Zubereitungen zunächst in eine für Lebens- und Genussmittel geeignete Matrix (Trägerstoff) eingearbeitet, z.B. in Form von Emulsionen, Liposomen, z.B. ausgehend von Phosphatidylcholin, Microsphären, Nanosphären oder auch in Kapseln, Granulaten oder Extrudaten. Besonders bevorzugt wird die Matrix dabei jeweils so gewählt, dass die Geschmack- und/oder Aromastoffe verzögert von der Matrix freigegeben werden, so dass eine langanhaltende Wirkung erzielt wird.

Bevorzugte Matrices, in welche die Geschmack- und/oder Aromastoffe vor deren Verwendung bei der Herstellung der erfindungsgemäßen Zubereitungen eingearbeitet werden, umfassen dabei vorzugsweise ein oder mehrere Materialien ausgewählt aus der folgenden Gruppe: Kohlenhydratpolymere (Polysaccharide) (z.B. Stärke, Stärkederivate, Cellulose oder Cellulosederivate (z.B. Hydroxypropylcellulose), Alginate, Gellan Gum, Agar oder Carragheen), natürliche Fette, natürliche Wachse (z.B. Bienenwachs, Carnaubawachs), Proteine, z.B. Gelatine, Komplexbildner (z.B. Cyclodextrine oder Cyclodextrinderivate, bevorzugt beta-Cyclodextrin).

Die Beladung der Matrices mit erfindungsgemäß einzusetzenden Geschmack- und/oder Aromastoffen kann, je nach Anforderung und gewünschtem sensorischen Profil, variieren. Üblicherweise liegt die Beladung an Geschmack- und/oder Aromastoffen im Bereich von 1 bis 60 Gew.-%, regelmäßig und vorzugsweise im Bereich 5 bis 40 Gew.-% bezogen auf das Gesamtgewicht von Matrix (Trägerstoff) und Geschmack- und/oder Aromastoffen.

Es hat sich ferner als vorteilhaft erwiesen die Geschmack- und/oder Aromastoffe vor deren Verwendung bei der Herstellung der erfindungsgemäßen Zubereitungen in eine sprühgetrocknete Form zu überführen. Als Matrices für die erfindungsgemäß einzusetzenden Geschmack- und/oder Aromastoffe in sprühgetrockneter Form können Einzelsubstanzen bzw. Substanzgemische eingesetzt werden. Vorteilhafte Trägerstoffe sind Kohlenhydrate und/oder Kohlenhydratpolymere (Polysaccharide). Als bevorzugte Trägerstoffe für die Geschmack- und/oder Aromastoffe in sprühgetrockneter Form sind zu nennen: Hydrokolloide wie Stärken, abgebaute Stärken, chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate (z.B. Na-Alginat), Pektin, Inulin oder Xanthan Gum zu nennen. Bevorzugte Trägerstoffe sind Maltodextrine sowie Mischungen von Maltodextrinen und Gummi Arabicum, wobei jeweils Maltodextrine mit DE-Werten im Bereich 15 bis 20 wiederum vorteilhaft sind. Der Zersetzungsgrad der Stärke wird mit der Kennzahl "Dextrose-Equivalent" (DE) gemessen, welche die Grenzwerte 0 für das langkettige Glucosepolymer und 100 für die reine Glucose annehmen kann. Die Einkapselung von Geschmack- und/oder Aromastoffen mittels Sprühtrocknung ist dem Fachmann bekannt, und beispielsweise in US 3,159,585, US 3,971,852, US 4,532,145 oder US 5,124,162 beschrieben. Sprühgetrocknete Aromen sind in vielen verschiedenen Geschmacksrichtungen und Partikelgrößen kommerziell erhältlich.

Geeignete Zuckeraustauschstoffe, die Bestandteil der erfindungsgemäßen Zubereitungen sein können, sind Zuckeralkohole wie beispielsweise Mannit, Sorbit und Sorbitsirup, Isomalt (z.B. Palatinit^{®}), Maltit und Maltitsirup, Lactit, Xylit, Erythrit, Leucrose, Arabinol, Arabitol, Adonitol, Alditol, Ducitol, Iditol, aber auch Fructooligosaccharide (z.B. Raftilose^{®}), Oligofructose oder Polydextrose.

Als typische Süßstoffe, die Bestandteil der erfindungsgemäßen Zubereitungen sein können, seien Saccharin (gegebenenfalls als Na-, K- oder Ca-Salz), Aspartam (z.B. NutraSweet^{®}) Cyclamat (gegebenenfalls als Na- oder Ca-Salz), Acesulfam-K (z.B. Sunett^{®}), Thaumatin oder Neohesperidin-Dihydrochalkon genannt. Ferner können auch andere Süßstoffe wie Steviosid, Rebaudiosid A, Glycyrrhizin, Ultrasüß, Osladin, Brazzein, Miraculin, Pentadin, Phyllodulcin, Dihydrochalcone, Arylharnstoffe, trisubstituierte Guanidine, Glycyrrhizin, Superaspartam, Suosan, Sucralose (Trichlorgalactosaccarose, TGS), Alitam, Monellin oder Neotame^{®} verwendet werden.

Bevorzugte scharf schmeckende und/oder den Speichelfluss im Mund anregende Substanzen und/oder ein Gefühl von Wärme und/oder ein kribbelndes Gefühl auf der Haut oder auf den Schleimhäuten hervorrufende Substanzen, die Bestandteil der erfindungsgemäßen Zubereitungen sein können, sind z.B.: Capsaicin, Dihydrocapsaicin, Gingerole, Paradole, Shogaole, Piperin, Carbonsäure-N-vanillylamide, insbesondere Nonansäure-N-vanillylamid, Pellitorin oder Spilanthol, 2-Nonensäureamide, insbesondere 2-Nonensäure-N-isobutylamid, 2-Nonensäure-N-4-hydroxy-3-methoxyphenylamid, Alkylether von 4-Hydroxy-3-methoxybenzylalkohol, insbesonders 4-Hydroxy-3-methoxybenzyl-n-butylether, Alkylether von 4-Acyloxy-3-methoxybenzylalkohol, insbesondere 4-Acetyloxy-3-methoxybenzyl-n-butylether und 4-Acetyloxy-3-methoxybenzyl-n-hexylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, (4-Hydroxy-3-methoxyphenyl)essigsäureamide, insbesondere (4-Hydroxy-3-methoxyphenyl)essigsäure-N-n-octylamid, Vanillomandelsäurealkylamide, Ferulasäurephenethylamide, Nicotinaldehyd, Methylnicotinat, Propylnicotinat, 2-Butoxyethylnicotinat, Benzylnicotinat, 1-Acetoxychavicol, Polygodial und Isodrimeninol, weiter bevorzugt cis- und/oder trans-Pellitorin gemäß WO 2004/000787 bzw. WO 2004/043906, Alkencarbonsäure-N-alkylamide gemäß WO 2005/044778, Mandelsäurealkylamide gemäß WO 03/106404 oder Alkyloxyalkansäureamide gemäß WO 2006/003210.

Bevorzugte scharf schmeckende und/oder ein Gefühl von Wärme und/oder eine kribbelndes Gefühl auf der Haut oder auf den Schleimhäuten hervorrufende natürliche Extrakte, die Bestandteil der erfindungsgemäßen Zubereitungen sein können, sind z.B.: Extrakte aus Paprika, Extrakte aus Pfeffer (z.B. Capsicum Extrakt),; Extrakte aus Chili-Pfeffer, Extrakte aus Ingwerwurzeln, Extrakte aus Aframomum melgueta, Extrakte aus Spilanthes-acmella, Extrakte aus Kaempferia galanga oder Extrakte aus Alpinia galanga.

Bevorzugte Stoffe zur Maskierung eines oder mehrerer unangenehmer Geschmackseindrücke, insbesondere eines bitteren, adstringierenden und/oder metallischen Geschmackseindrucks oder Nachgeschmacks, die Bestandteil der erfindungsgemäßen Zubereitungen sein können, sind: Lactisol [20-(4-Methoxyphenyl)milchsäure] (vgl. US 5,045,336), 2,4-Dihydroxybenzoesäure-Kaliumsalz (vgl. US 5,643,941), Ingwerextrakten (vgl. GB 2,380,936), Neohesperidindihydrochalcon (vgl. Manufacturing Chemist 2000, Juli-Heft, S. 16-17), bestimmte Flavone (2-Phenylchrom-2-en-4-one) (vgl. US 5,580,545), bestimmte Nucleotide, wie beispielsweise Cytidin-5'-monophosphate (CMP) (vgl. US 2002/0177576), bestimmte Natriumsalze wie Natriumchlorid, Natriumcitrat, Natriumacetat und Natriumlactat (vgl. Nature, 1997, Bd. 387, S. 563), ein Lipoprotein aus β-Lactoglobulin und Phosphatidinsäure (vgl. EP-A 635 218), Neodiosmin [5,7-Dihydroxy-2-(4-methoxy-3-hydroxyphenyl)-7-O-neohesperidosyl-chrom-2-en-4-on] (vgl. US 4,154,862), vorzugsweise Hydroxyflavanone gemäß EP 1 258 200, dabei wiederum bevorzugt 2-(4-Hydroxyphenyl)-5,7-dihydroxychroman-4-on (Naringenin), 2-(3,4-Dihydroxyphenyl)-5,7-dihydroxychroman-4-on (Eriodictyol), 2-(3,4-Dihydroxyphenyl)-5-hydroxy-7-methoxychroman-4-on (Eriodictyol-7-methylether), 2-(3,4-Dihydroxyphenyl)-7-hydroxy-5-methoxychroman-4-on (Eriodictyol-5-methylether) und 2-(4-Hydroxy-3-methoxyphenyl)-5,7-dihydroxychroman-4-on (Homoeriodictyol), deren (2S)- oder (2R)-Enantiomere oder Gemische derselben sowie deren ein- oder mehrwertigen Phenolatsalze mit Na⁺, K⁺, NH₄⁺' Ca²⁺, Mg²⁺ oder Al³⁺ als Gegenkationen, oder γ-Aminobuttersäure (4-Aminobutansäure, als neutrale Form ("inneres Salz") oder in der Carboxylat- oder Ammoniumform) gemäß WO 2005/096841.

Stoffe, die bitter, adstringierend, pappig, staubig, trocken, mehlig, ranzig oder metallisch schmecken, sind beispielsweise: Xanthinalkaloide Xanthine (Coffein, Theobromin, Theophyllin), Alkaloide (Chinin, Brucin, Nicotin), phenolische Glycoside (z.B. Salicin, Arbutin), Flavonoidglycoside (z.B. Hesperidin, Naringin), Chalcone und Chalconglycoside, hydrolisierbare Tannine (Gallus- oder Elagsäureester von Kohlenhydraten, z.B. Pentagalloylglucose), nichthydrolysierbare Tannine (ggfs. galloylierte Catechine oder Epicatechine und deren Oligomere, z.B. Proanthyocyanidine oder Procyanidine, Thearubigenin), Flavone (z.B. Quercetin, Taxifolin, Myricetin), andere Polyphenole (γ-Oryzanol, Kaffeesäure oder deren Ester), terpenoide Bitterstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (Kaliumchlorid, Natriumsulfat, Magnesiumsulfat), bestimmte pharmazeutische Wirkstoffe (z.B. Fluorchinolon-Antibiotika, Paracetamol, Aspirin, beta-Lactam-Antibiotika, Ambroxol, Propylthiouracil [PROP], Guaifenesin), bestimmte Vitamine (beispielsweise Vitamin H, Vitamine aus der B-Reihe wie Vitamin B1, B2, B6, B12, Niacin, Panthotensäure), Denatoniumbenzoat, Sucraloseoctaacetat, Kaliumchlorid, Magnesiumsalze, Eisensalze, Aluminiumsalze, Zinksalze, Harnstoff, ungesättigte Fettsäuren, insbesondere ungesättigte Fettsäuren in Emulsionen, Aminosäuren (z.B. Leucin, Isoleucin, Valin, Tryptophan, Prolin, Histidin, Tyrosin, Lysin und Phenylalanin), Peptide (insbesondere Peptide mit einer Aminosäure aus der Gruppe Leucin, Isoleucin, Valin, Tryptophan, Prolin oder Phenylalanin am N- oder C-Terminus).

Stoffe, die einen bitteren, adstringierenden, pappigen, staubigen, trockenen, mehligen, ranzigen oder metallischen Nachgeschmack haben, können beispielsweise zur Gruppe der Süßstoffe oder der Zuckeraustauschstoffe gehören. Beipielsweise seien genannt: Aspartam, Neotam, Superaspartam, Saccharin, Sucralose, Tagatose, Monellin, Stevioside, Thaumatin, Miraculin, Glycerrhizin und deren Derivate, Cyclamat und die pharmazeutisch akzeptablen Salze der vorgenannten Verbindungen.

Vorteilhafte Zusatzstoffe zur Einarbeitung in die erfindungsgemäßen Zubereitungen sind Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), Pflanzenextrakte, natürliche oder synthetische Farbstoffe oder Farbpigmente (z.B. Carotinoide, Flavonoide, Anthocyane, Chlorophyll und deren Derivate).

Erfindungsgemäße Zubereitungen können ferner Antioxidantien enthalten bzw. Stoffe, die eine antioxidative Wirkung verstärken können, vorzugsweise natürlich vorkommende Tocopherole und deren Derivate (z.B. Vitamin E - acetat), Vitamin C und ihre Salze bzw. Derivate (z.B. Ascorbylpalmitat, Mg - Ascorbylphosphat, Ascorbylacetat), Vitamin A und Derivate (Vitamin A - palmitat), Tocotrienole, Flavonoide, alpha-Hydroxysäuren (z.B. Citronensäure, Milchsäure, Apfelsäure, Weinsäure) und deren Na-, Ka- und Ca-Salze, Flavonoide, Quercetin, phenolische Benzylamine, Propylgallat, Octylgallat, Dodecylgallat, Butylhydroxyanisol (BHA, E320), Butylhydroxytoluol (BHT, 2,6-Di-tert.-butyl-4-methyl-phenol, E321), Lecithine, Mono- u nd Diglyceride von Speisefettsäuren verestert mit Citronensäure, Carotinoide, Carotine (z.B. α-Carotin, β-Carotin, Lycopin) und deren Derivate, Phytinsäure, Lactoferrin, EDTA, EGTA), Folsäure und deren Derivate, Ubichinon und Ubichinol und deren Derivate, Ferulasäure und deren Derivate, Zink und dessen Derivate (z.B. ZnO, ZnSO₄), Selen und dessen Derivate (z.B. Selenmethionin),Orthophosphate und Na-, Ka- und Ca-Salze der Monophosphorsäure sowie aus Pflanzen isolierte Inhaltsstoffe, Extrakte bzw. Fraktionen davon z.B. aus Tee, Grüntee, Algen, Traubenkernen, Weizenkeimen, Kamille, Rosmarin, Oregano.

Die erfindungsgemäßen Zubereitungen können beispielsweise folgende Farbstoffe, Färbemittel oder Pigmente enthalten: Lactoflavin (Riboflavin), beta-Carotin, Riboflavin-5'-phosphat, alpha-Carotin, gamma-Carotin, Cantaxanthin, Erythrosin, Kurkumin, Chinolingelb, Gelborange S, Tartrazin, Bixin, Norbixin (Annatto, Orlean), Capsanthin, Capsorubin, Lycopin, beta-Apo-8'-Carotinal, beta-Apo-8'-Carotinsäureethylester, Xantophylle (Flavoxanthin, Lutein, Kryptoxanthin, Rubixanthin, Violaxanthin, Rodoxanthin), Echtes Karmin (Karminsäure, Cochenille), Azorubin, Cochenillerot A (Ponceau 4 R), Beetenrot, Betanin, Anthocyane, Amaranth, Patentblau V, Indigotin I (Indigo-Karmin), Chlorophylle, Kupferverbindungen der Chlorophylle, Brillantsäuregrün BS (Lisamingrün), Brillantschwarz BN, Carbo medicinalis vegetabilis, Titandioxid, Eisenoxide und - hydroxide, Calciumcarbonat, Aluminium, Silber, Gold, Rubinpigment BK (Litholrubin BK), Methylviolett B, Viktoriablau R, Viktoriablau B, Acilanbrillantblau FFR (Brillantwollblau FFR), Naphtolgrün B, Acilanechtgrün 10 G (Alkaliechtgrün 10 G), Ceresgelb GRN, Sudanblau II, Ultramarin, Phtalocyaninblau, Phtalocayaningrün, Echtsäureviolett R. Es können weitere, natürlich gewonnene Extrakte (z.B. Paprikaextrakt, Schwarzmöhrenextrakt, Rotkohlextrakt) zu Färbezwecken verwendet werden. Gute Ergebnisse wurden auch erzielt mit den im Folgenden genannten Farben, den sogenannten Aluminium Lakes: FD & C Yellow 5 Lake, FD & C Blue 2 Lake, FD & C Blue 1 Lake, Tartrazine Lake, Quinoline Yellow Lake, FD & C Yellow 6 Lake, FD & C Red 40 Lake, Sunset Yellow Lake, Carmoisine Lake, Amaranth Lake, Ponceau 4R Lake, Erythrosyne Lake, Red 2G Lake, Allura Red Lake, Patent Blue V Lake, Indigo Carmine Lake, Brilliant Blue Lake, Brown HT Lake, Black PN Lake, Green S Lake und deren Mischungen.

Geeignete (mineralische) Füllstoffe zur Einarbeitung in die erfindungsgemäßen Zubereitungen sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen.

In einer vorteilhaften Ausgestaltung enthalten die erfindungsgemäßen Zubereitungen Zusatzstoffe, wie sie auch in Mundhygieneprodukten oder Zahnpflegemitteln verwendet werden, dabei vorzugsweise mindestens einen Zusatzstoff ausgewählt aus folgender Gruppe: Abrasiva (Schleif- oder Poliermittel), wie z.B. Kieselsäuren, Calciumcarbonate, Calciumphosphate, Alumiuniumoxide und/oder Hydroxylapatite, und/oder oberflächenaktive Substanzen wie z.B. Natriumlaurylsulfat, Natriumlaurylsarcosinat und/oder Cocamidopropylbetain, und/oder Feuchthaltemittel wie z.B. Glycerin und/oder Sorbit, Süßstoffe wie z.B. Saccharin, Geschmackskorrigenzien für unangenehme Geschmackseindrücke, Geschmackskorrigenzien für in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Carboxymethylcellulose, Polyethylenglycole, Carrageenan und/oder Laponite^{®}, Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid und/oder Natriumbicarbonat.

Vorzugsweise enthält eine erfindungsgemäße Zubereitung neben einer oder mehrerer Verbindungen der Formel 1 oder der Formel 1A zusätzlich einen oder mehrere Stoffe zur Verbesserung der Mundhygiene, wie beispielsweise Stoffe zu Bekämpfung oder Verhinderung von Plaque, Zahnstein oder Karies sowie weitere zur Bekämpfung oder Verhinderung von Mundgeruch. Es sei in diesem Zusammenhang auf die US 5,043,154 hingewiesen. Beispielhaft seien genannt Zn-Salze, wie Zn-citrat, Zn-fluorid, Sn-Salze, wie Sn-fluoride, Cu-Salze, Fluoride, z.B. Aminfluoride, Alkalifluoride wie Na-fluorid, Erdalkalifluoride, Ammoniumfluorid, Phosphate, Pyrophosphate, Fluorphosphate, wie Na-monofluorphosphat, Al-mono- und Al-difluorphosphat, alpha-lonon, Geraniol, Thymol, Isomenthylacetat, Panthenol (Provitamin B5), Xylitol, Allantoin, Niacinamid (Vitamin B3), Tocopherylacetat (Vitamin E - Actetat), Poloxamer.

Eine erfindungsgemäße Zubereitung kann neben einer oder mehrerer Verbindungen der Formel 1 oder der Formel 1A zusätzlich einen oder mehrere weitere antimikrobielle Wirkstoffe zur Verbesserung der Mundhygiene enthalten. Diese antimikrobiellen Wirkstoffe können hydrophiler, amphoterer oder hydrophober Natur sein. Bevorzugte weitere antimikrobielle Wirkstoffe sind: Triclosan, Chlorhexidin und dessen Salze (z.B. dessen -acetat, -gluconat, oder - hydrochlorid), Peroxide, Phenole und deren Salze, Domiphenbromid (Phenododeciniumbromid), Bromchlorophen, Zn-Salze, Chlorophylle, Cu-Salze, Cu-Gluconat, Cu-Chlorophyll, Natriumlaurylsulfat, quarternäre Monoammonium Salze wie Cocoalkylbenzyldimethylammoniumchlorid oder auch Pyridiniumsalze wie Cetylpyridiniumchlorid. Neben Einzelwirkstoffen können Mischungen von Wirkstoffen oder natürliche Extrakte bzw. Fraktionen hiervon enthaltend Wirkstoffe eingesetzt werden, wie z.B. solche erhältlich aus Neem, Berberitze, Fenchel, Grüntee, Ringelblume, Kamille, Rosmarin, Thymian, Propolis oder Gelbwurz.

Erfindungsgemäße Zubereitungen, die für den Einsatz als Zahn- und/oder Mundpflegeprodukt vorgesehen sind, sind frei von kariogenen Substanzen, insbesondere frei von Saccharose, Glucose, Lactose, hydrolisierter Lactose, Sorbose, Arabinose, Xylose, Mannose, Maltose, Galactose, Maltotriose und Fructose.

Die nachfolgenden Beispiele sollen die vorliegende Erfindung verdeutlichen, ohne sie einzuschränken. Sofern nicht anders angegeben, beziehen sich alle Angaben auf das Gewicht.

### Beispiele:

### Beispiel 1: In-vitro Test zur Mundgeruchsreduktion

Der Test beruht auf der Arbeit von Goldberg und Rosenberg (Production of Oral Malodor in an in vitro System, S. Goldberg and M. Rosenberg, pp.143 - 150, in: Bad Breath- A multidisciplinary Approach, Eds: D. van Steenberghe, M. Rosenberg, Leuven University Press, 1996) und wurde zur besseren Reproduzierbarkeit angepaßt.

Ein steriles Flüssigmedium, das mit frischem Morgenspeichel angeimpft wird, wird für einige Tage bei 37 °C bebrütet und anschließend von einem Prüferpanel abgerochen.

Es hat sich ein intensiver, typischer Mundgeruch gebildet. Nicht angeimpfte Kontrollen haben nur einen schwachen Mediengeruch.

Als Kontrolle für die Tests wurde Triclosan^{®} in einer Konzentration von 0,05 % dazugegeben. Die angeimpften Kolben hatten nach der Inkubation den gleichen Geruch wie die nicht angeimpften Kolben.

Die Verwendung von typischen Aromastoffen für Oral Care Anwendungen, zeigte bei Einsatz von 0,1% Konzentrationen im Test zumeist einen sehr unangenehmen Mischgeruch, der aus der Mischung des Mundgeruchs mit dem Aromastoff entstand. Teilweise war der Aromastoff nicht mehr wahrnehmbar, da er offenbar durch die Mikroorganismen des Speichels abgebaut wurde.

Bei Einsatz von 0,0025 Gew.-% 2-(Benzoylamino)benzoesäure (Verbindung 27) konnte kein Geruch analog zu 0,05 Gew.-% Triclosan^{®} festgestellt werden. Bei 0,001 Gew.-% war ein nicht unangenehmer Geruch wahrnehmbar, der keine Ähnlichkeit mit Mundgeruch hatte.

Die minimalen Wirkkonzentrationen verschiedener Verbindungen der Formel 1 oder der Formel 1A, bei denen kein Geruch festgestellt werden konnte, sind nachfolgend dargestellt:

| | |
|---|---|
| 2-[(3-Hydroxybenozyl)amino]benzoesäure (Verbindung 28) | 0,0005 % (= 5 ppm) |
| 2-{[(2E)-3-Phenylprop-2-enoyl]amino}benzoesäure (Verbindung 102) | 0,001 % (= 10 ppm) |
| 2-[(4-Hydroxybenzoyl)amino]benzoesäure (Verbindung 24) | 0,001 % |
| 2-[(4-Hydroxy-3-methoxybenzoyl)amino]benzoesäure (Verbindung 25) | 0,001 % |
| 2-{[(2E)-3-(4-Hydroxyphenyl)prop-2-enoyl]amino}benzoesäure (Verbindung 10) | 0,0025 % (= 25 ppm) |
| 5-Hydroxy-2-{[(2E)-3-phenylprop-2-enoyl]amino}benzoesäure (Verbindung 12) | 0,0025 % |
| 2-{[(2E)-3-(4-methoxyphenyl)prop-2-enoyl]amino}benzoesäure (Verbindung 101) | 0,0025 % |
| 2-(Benzylamino)benzoesäure (Verbindung 27) | 0,0025 % |
| 2-[(2-Hydroxybenzoyl)amino]benzoesäure (Verbindung 29) | 0,0025 % |
| 2-[(2,4-Dihydroxybenzoyl)amino]benzoesäure (Verbindung 26) | 0,0025 % |
| 2-[(3-Hydroxy-4-methoxybenzoyl)amino]benzoesäure (Verbindung 23) | 0,0025 % |
| 5-Hydroxy-2-{[(2E)-3-(3,4-dihydroxyphenyl)prop-2-enoyl]amino}benzoesäure (Verbindung 3) | 0,0025 % |
| Salicylanilid (Verbindung 100) | 0,0025 % |
| 5-Hydroxy-2-{[(2E)-3-(4-hydroxyphenyl)prop-2-enoyl]amino}benzoesäure (Verbindung 7) | 0,005 % (= 50 ppm) |
| 2-{[(2E)-3-(4-Hydroxy-3-methoxyphenyl)prop-2-enoyl]amino}benzoesäure (Verbindung 9) | 0,005 % |
| 2-{[(2E)-3-(3,4-Dihydroxyphenyl)prop-2-enoyl]amino}benzoesäure (Verbindung 2) | 0,005 % |
| 2-{[3-(4-Hydroxyphenyl)propanoyl]amino}benzoesäure (Verbindung 8) | 0,005 % |
| 2-[(3,4-Dihydroxybenzoyl)amino]benzoesäure (Verbindung 20) | 0,005 % |
| 5-Hydroxy-2-{[(2E)-3-(4-hydroxy-3-methoxyphenyl)prop-2-enoyl]amino}benzoesäure (Verbindung 4) | 0,01 % (= 100 ppm) |
| 5-Hydroxy-2-{[(2E)-3-(3,4-Dimethoxyphenyl)prop-2-enoyl]amino}benzoesäure (Verbindung 13) | 0,01 % |
| 4-Hydroxy-2-[(4-hydroxybenzoyl]amino}benzoesäure (Verbindung 75) | 0,01 % |

Andere als antimikrobiell wirksam bekannte Verbindungen wie Eugenol und Thymol unterdrückten bei 0,1 % Einsatzkonzentration ebenfalls die Mundgeruchsbildung in diesem Test, hatten aber den Nachteil des sehr deutlichen Eigengeruchs.

### Beispiel 2: Bestimmunq der minimalen Hemmkonzentration

Die minimale Hemmkonzentration (MHK) wurde exemplarisch für 2-(Benzoylamino)benzoesäure (Verbindung 27) im Reihenverdünnungstest gegen verschiedene Mundkeime bestimmt. Das Ergebnis ist in folgender Tabelle dargestellt:

| Organismus | MHK [ppm] | Typ |
|---|---|---|
| Fusobacterium nucleatum | 500 | bakteriostatisch |
| Fusobacterium nucleatum | 1000 | bakterizid |
| Prevotella intermedia | 500 | bakteriostatisch |
| Prevotella intermedia | 1000 | bakterizid |
| Staphylococcus aureus | 500 | bakterizid |
| Veillonella parvula | 250 | bakterizid |

Es wurde also eine bakterizide Wirkung gegen die Mundgeruch verursachenden Keime Fusobacterium nucleatum und Prevotella intermedia nachgewiesen.

Gegenüber den Keimen Candida albicans, Aspergillus niger oder auch Escherichia coli, welche nicht im Zusammenhang mit Mundgeruch stehen, zeigte beispielsweise die erfindungsgemäß einzusetzende Verbindung 10 (Avenanthramid D) bei einer Dosierung von 1000 ppm keine Wirkung.

### Formulierungsbeispiele

### F1: Gel-Zahncreme mit Wirksamkeit gegen Mundgeruch

| | I(%) | II(%) | III(%) |
|---|---|---|---|
| Na-carboxymethylcellulose | 0,40 | 0,40 | 0,40 |
| Sorbitol 70 %, in Wasser | 72,00 | 72,00 | 72,00 |
| Polyethylenglykol (PEG) 1500 | 3,00 | 3,00 | 3,00 |
| Na-saccharinat | 0,07 | 0,07 | 0,07 |
| Na-fluorid | 0,24 | 0,24 | 0,24 |
| p-Hydroxybenzoesäure (PHB)-Ethylester | 0,15 | 0,15 | 0,15 |
| Aroma | 1,0 | 1,00 | 1,00 |
| 2-[(3-Hydroxybenozyl)amino]benzoesäure (Verbindung 28) | 0,025 | 0,06 | 0,10 |
| Abrasivkieselsäure | 11,00 | 11,00 | 11,00 |
| Verdickungskieselsäure | 6,00 | 6,00 | 6,00 |
| Natriumdodecylsulfat (SDS) | 1,40 | 1,40 | 1,40 |
| Wasser dest. | Ad 100,00 | Ad 100,00 | Ad 100,00 |

### F2: Zahncreme gegen Plaque mit Wirksamkeit gegen Mundgeruch

| | I(%) | II (%) | III (%) |
|---|---|---|---|
| Na-carboxymethylcellulose | 1,00 | 1,00 | 1,00 |
| Glycerin | 12,50 | 12,50 | 12,50 |
| Sorbitol 70 %, in Wasser | 29,00 | 29,00 | 29,00 |
| Na-saccharinat | 0,20 | 0,20 | 0,20 |
| Na-fluorid | 0,22 | 0,22 | 0,22 |
| Azacycloheptan-2,2-diphosphosäure, Di-natriumsalz | 1,00 | 1,00 | 1,00 |
| Bromchlorophen | 0,10 | 0,10 | 0,10 |
| Pfefferminz-Aroma | 1,10 | 1,10 | 1,10 |
| Salicylanilid (Verbindung 100) | 0,025 | 0,08 | 0,15 |
| Abrasivkieselsäure | 15,00 | 15,00 | 15,00 |
| Verdickungskieselsäure | 5,00 | 5,00 | 5,00 |
| Natriumdodecylsulfat (SDS) | 1,50 | 1,50 | 1,50 |
| Wasser dest. | Ad 100,00 | Ad 100,00 | Ad 100,00 |

### F3: Zahncreme gegen Plaque mit Wirksamkeit gegen Mundgeruch

| | I (%) | II (%) | III (%) |
|---|---|---|---|
| Carragenan | 0,90 | 0,90 | 0,90 |
| Glycerin | 15,00 | 15,00 | 15,00 |
| Sorbitol 70 %, in Wasser | 25,00 | 25,00 | 25,00 |
| PEG 1000 | 3,00 | 3,00 | 3,00 |
| Na-fluorid | 0,24 | 0,24 | 0,24 |
| Tetrakalium-diphosphat | 4,50 | 4,50 | 4,50 |
| Tetranatrium-diphosphat | 1,50 | 1,50 | 1,50 |
| Na-saccharinat | 0,40 | 0,40 | 0,40 |
| Fällungskieselsäure | 20,00 | 20,00 | 20,00 |
| Titandioxid | 1,00 | 1,00 | 1,00 |
| PHB-Methylester | 0,10 | 0,10 | 0,10 |
| Spearmint-Aroma | 1,10 | 1,10 | 1,10 |
| 2-(Benzylamino)benzoesäure (Verbindung 27) | 0,025 | 0,06 | 0,10 |
| Natriumdodecylsulfat | 1,30 | 1,30 | 1,30 |
| Wasser dest. | Ad 100,00 | Ad 100,00 | Ad 100,00 |

### F4: Zahncreme gegen empfindliche Zähne mit Wirksamkeit gegen Mundgeruch

| | I(%) | II(%) | III(%) |
|---|---|---|---|
| Na-carboxymethylcellulose | 0,70 | 0,70 | 0,70 |
| Xanthan Gum | 0,50 | 0,50 | 0,50 |
| Glycerin | 15,00 | 15,00 | 15,00 |
| Sorbitol 70 %, in Wasser | 12,00 | 12,00 | 12,00 |
| K-nitrat | 5,00 | 5,00 | 5,00 |
| Na-monofluorphosphat | 0,80 | 0,80 | 0,80 |
| PHB-Methylester | 0,15 | 0,15 | 0,15 |
| PHB-Propylester | 0,05 | 0,05 | 0,05 |
| Na-saccharinat | 0,20 | 0,20 | 0,20 |
| Aroma | 1,00 | 1,00 | 1,00 |
| 2-(Benzylamino)benzoesäure (Verbindung 27) | 0,025 | 0,06 | 0,10 |
| Ca-carbonat | 35,00 | 35,00 | 35,00 |
| Siliciumdioxid | 1,00 | 1,00 | 1,00 |
| Natriumdodecylsulfat (SDS) | 1,50 | 1,50 | 1,50 |
| Wasser dest. | Ad 100,00 | Ad 100,00 | Ad 100,00 |

### F5: Zahncreme gegen empfindliche Zähne mit Wirksamkeit gegen Mundgeruch

| | I (%) | II (%) | III (%) |
|---|---|---|---|
| Hydroxyethylcellulose | 1,40 | 1,40 | 1,40 |
| Guar Gum | 0,60 | 0,60 | 0,60 |
| Glycerin | 18,00 | 18,00 | 18,00 |
| Sorbitol 70 %, in Wasser | 12,00 | 12,00 | 12,00 |
| Na-Saccharinat | 0,35 | 0,35 | 0,35 |
| Farbstoff | 0,01 | 0,01 | 0,01 |
| PHB-Methylester | 0,15 | 0,15 | 0,15 |
| PHB-Propylester | 0,04 | 0,04 | 0,04 |
| Sr-chlorid | 10,50 | 10,50 | 10,50 |
| Zimtaroma | 1,20 | 1,20 | 1,20 |
| 2-[(3-Hydroxy-4-methoxybenzoyl)amino]benzoesäure (Verbindung 23) | 0,025 | 0,10 | 0,18 |
| Fällungskieselsäure | 15,00 | 15,00 | 15,00 |
| Siliciumdioxid | 1,60 | 1,60 | 1,60 |
| Natriumdodecylsulfat | 1,30 | 1,30 | 1,30 |
| Wasser dest. | Ad 100,00 | Ad 100,00 | Ad 100,00 |

### F6: Gebrauchsfertiges Mundwasser mit Fluorid und Wirksamkeit gegen Mundgeruch

| | I (%) | II (%) | III (%) |
|---|---|---|---|
| Ethanol | 7,00 | 7,00 | 7,00 |
| Glycerin | 12,00 | 12,00 | 12,00 |
| Na-fluorid | 0,05 | 0,05 | 0,05 |
| Pluronic F-127^{®} (BASF, oberflächenaktive Substanz) | 1,40 | 1,40 | 1,40 |
| Na-phosphatpuffer pH 7,0 | 1,10 | 1,10 | 1,10 |
| Sorbinsäure | 0,20 | 0,20 | 0,20 |
| Na-saccharinat | 0,10 | 0,10 | 0,10 |
| Pfefferminz-Aroma | 0,15 | 0,15 | 0,15 |
| 2-(Benzylamino)benzoesäure (Verbindung 27) | 0,01 | 0,02 | 0,03 |
| Farbstoff | 0,01 | 0,01 | 0,01 |
| Wasser dest. | Ad 100,00 | Ad 100,00 | Ad 100,00 |

### F7: Mundwasserkonzentrat mit Wirksamkeit gegen Mundgeruch

| | I (%) | II (%) | III (%) |
|---|---|---|---|
| Ethanol, 95%ig | 80,00 | 80,00 | 80,00 |
| Na-cyclamat | 0,15 | 0,15 | 0,15 |
| Zimt-Aroma | 3,50 | 3,50 | 3,50 |
| Farbstoff | 0,01 | 0,01 | 0,01 |
| 2-{[(2E)-3-Phenylprop-2-enoyl]amino}benzoesäure (Verbindung 102) | 0,50 | 1,0 | 3,0 |
| Wasser dest. | Ad 100,00 | Ad 100,00 | Ad 100,00 |

### F8: Kaugummi gegen Mundgeruch

| | I (%) | II (%) | III (%) |
|---|---|---|---|
| Kaugummibase | 21,00 | 21,00 | 21,00 |
| Glucose Sirup | 16,50 | 16,50 | 16,50 |
| Glycerin | 0,50 | 0,50 | 0,50 |
| Puderzucker | 60,45 | 60,40 | 60,30 |
| Spearmint-Aroma | 1,50 | 1,50 | 1,50 |
| 2-(Benzylamino)benzoesäure (Verbindung 27) | 0,05 | 0,15 | 0,35 |

### F9: Zuckerfreier Kaugummi gegen Mundgeruch

| | I (%) | II (%) | III (%) |
|---|---|---|---|
| Kaugummibase | 30,00 | 30,00 | 30,00 |
| Sorbit, Pulver | 38,45 | 38,40 | 38,30 |
| Palatinit | 9,50 | 9,50 | 9,50 |
| Xylit | 2,00 | 2,00 | 2,00 |
| Mannit | 3,00 | 3,00 | 3,00 |
| Aspartame | 0,10 | 0,10 | 0,10 |
| Acesulfam K | 0,10 | 0,10 | 0,10 |
| Emulgum / Emulgator | 0,30 | 0,30 | 0,30 |
| Sorbitol 70 %, in Wasser | 14,00 | 14,00 | 14,00 |
| Glycerin | 1,00 | 1,00 | 1,00 |
| Zimt-Menthol-Aroma | 1,50 | 1,50 | 1,50 |
| 2-(Benzylamino)benzoesäure (Verbindung 27) | 0,05 | 0,10 | 0,20 |

### F10: Gelatinekapsel gegen Mundgeruch zum Direktverzehr

| | I (%) | II (%) | III (%) |
|---|---|---|---|
| Gelatinehülle: | | | |
| Glycerin | 2,014 | 2,014 | 2,014 |
| Gelatine 240 Bloom | 7,91 | 7,91 | 7,91 |
| Sucralose | 0,065 | 0,065 | 0,065 |
| Allura Rot | 0,006 | 0,006 | 0,006 |
| Brillantblau | 0,005 | 0,005 | 0,005 |
| | | | |
| Kernzusammensetzung: | | | |
| Pflanzenöl Triglycerid | 82,0 | 74,0 | 60,0 |
| Aroma B | 7,75 | 15,0 | 25,0 |
| 2-(Benzylamino)benzoesäure (Verbindung 27) | 0,25 | 1,0 | 5,0 |

| | | | |
|---|---|---|---|
| Aroma B hatte dabei folgende Zusammensetzung (Angaben jeweils in Gew.-%): 0,1% Neotam Pulver, 0,05% Aspartam, 29,3% Pfefferminzöl arvensis, 29,3% Pfefferminz piperita Öl Willamette, 2,97% Sucralose, 2,28% Triacetin, 5,4% Diethyltartrat, 12,1% Pfefferminzöl yakima, 0,7% Ethanol, 3,36% 2-Hydroxyethylmenthylcarbonat, 3,0% 2-Hydroxypropylmenthylcarbonat, 0,27% Vanillin, 5,5% D-Limonen, 5,67% L-Menthylacetat. | | | |

Die zum Direktverzehr geeignete Gelatinekapsel hatte einen Durchmesser von 5 mm, das Gewichtsverhältnis von Kernmaterial zu Hüllmaterial lag bei 90 : 10. Die Kapsel öffnete sich im Mund innerhalb von weniger als 10 Sekunden und löste sich vollständig innerhalb von weniger als 50 Sekunden auf.

Die synergistische Verstärkung der Juckreiz lindernden Wirksamkeit der erfindungsgemäßen Wirkstoffkombinationen enthaltend (a) Verbindung(en) der Formel 1A und (b) ein oder mehrere Kühlwirkstoffe geht aus den im folgenden beschriebenen humanen in vivo-Studien ("Skin Prick Test") hervor.

Beispiel 3: Humaner ,,Skin Prick Test" zum Nachweis der synergistisch verstärkten Wirksamkeit von Kombinationen bestehend aus einem Juckreiz lindernden Wirkstoff (Dihydroavenanthramid D; CARN: 697235-49-7; Benzoic acid, 2-[[3-(4-hydroxyphenyl)-1-oxopropyl]amino]- (9Cl) ) und einem Kühlwirkstoff Menthyl lactat (Handelsname: Frescolat®ML) 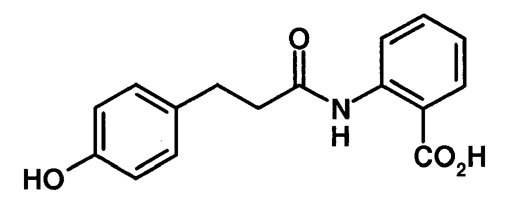 Strukturformel: Dihydroavenanthramid D (= Verbindung 8)

### Beschreibung des Testmethode:

Die Untersuchungen wurden an 10 Probanden durchgeführt (5 Testareale auf der Unterarm-Innenseite; 1 x unbehandelt + 4 Applikationsflächen für 4 Proben)

### Proben:

1. Placeboformulierung
2. Produkt A: wie Placeboformulierung, zusätzlich enthaltend 1 Gew.-% Menthyl lactat;
3. Produkt B: wie Placeboformulierung, zusätzlich enthaltend 0,05 Gew.-% Dihydroavenanthramid D;
4. Produkt C: wie Placeboformulierung, zusätzlich enthaltend 0,025 Gew.-% Dihydroavenanthramid D und 0,5 Gew.-% Frescolat ML Versuchsdurchführung:

Eine definierte Menge einer Histaminchlorid-Lösung (HAL Allergie GmbH, Düsseldorf; Konzentration: 10mg/ml) wurde auf die Haut der Unterarm-Innenseite appliziert. Anschließend wurde die Haut mit einer speziellen Lanzette (Feather Safety Razor; LTD Medical Division, Japan) oberflächlich leicht angeritzt. Hierdurch wurde innerhalb von 5 Minuten ein starker Juckreiz erzeugt. Anschließend wurden die Placeboformulierung sowie die Produkte A-C appliziert (Menge: 2mg/cm²). Der Einfluß der Produkte A-C auf die Juckreiz-Linderung relativ zum unbehandelten Areal und zum Placebo wurde nach 90' ermittelt. Die Versuchsdurchführung erfolgte hierbei unter standardisierten Bedingungen (20°C +/- 1 %; Luftfeuchtigkeit: 50% +/- 5%).

### Ergebnis:

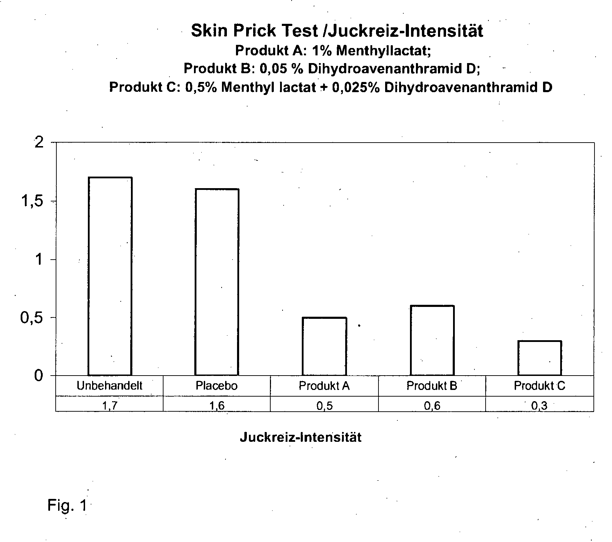

1. Die Produkte A-C führten gegenüber dem unbehandelten Areal und gegenüber der Placeboformulierung ohne Wirkstoff zu einer signifikant stärkeren Reduktion des Juckreizes (Abb. 1)
2. Produkt C zeigte gegenüber den Produkten A und B die stärkste Reduktion des Juckreizes (Abb. 1).

Die humane in vivo-Skin Prick Test-Studie belegt exemplarisch, daß durch die Kombination von Anthranilsäureamiden der allg. Formel 1A mit Kühlwirkstoffen eine stärkere Reduktion des Juckreizes erreicht wird. Für Produkt C konnte im Vergleich zu den Produkten A und B eine signifikant stärkere Juckreiz-Reduktion beobachtet werden (Fig. 1). Die Reduktion des Juckreizes übertraf dabei die rein additiv zu erwartenden Reduktionswerte, wodurch ein synergistischer Effekt des Produktes C enthaltend 0,5 Gew.-% Frescolat ML und 0,025 Gew.-% Dihydroavenanthramid D (Verbindung 8) eindeutig erwiesen ist. Eine synergistische Verstärkung der Rötungs-Reduktion von Produkt C gegenüber den Produkten A und B konnte im Rahmen des Experiments ebenfalls nachgewiesen werden.

Die synergistische Wirksamkeitsverstärkung der erfindungsgemäßen Wirkstoffkombination bestehend aus einem Kühlwirkstoff und einem Juckreiz-lindernden Wirkstoff lässt sich basierend auf den vorliegenden sensorischen Daten anhand der Gleichung von Kull (F.C. Kull et al.; Applied Microbiology Vol. 9, p. 538-541 (1961); David C. Steinberg; Cosmetics & Toiletries Vol. 115 (No. 11), p. 59-62; November 2000; zur Berechnungsmethode siehe auch Tabelle 10) nachweisen. Die Kull'sche Gleichung gestattet es, die Reinsubstanzen und die daraus hergestellten Wirkstoffmischungen hinsichtlich Ihrer Juckreiz-lindernden Wirksamkeit zu vergleichen. Bestimmt wird hierbei der sogenannte Synergie-Index (SI), der ein Maß für eine synergistische, aber auch für eine eventuell antagonistische Wirksamkeit eines Juckreiz lindernden Gemisches darstellt. Ein synergistischer Effekt ist evident, wenn der ermittelte SI-Wert kleiner 1 ist. Errechnet sich ein SI von exakt 1 liegt hingegen ein rein additiver Effekt von zwei Juckreiz-lindernden Produkten vor. Bei einem SI-Wert größer als 1 liegt hingegen ein (häufig unerwünschter) antagonistischer Effekt vor.

Nachfolgend ist beispielhaft die Berechnung des SI-Wertes für die Behandlung mit einer Mischung bestehend aus Menthyl lactat und Dihydroavenanthramid D nach einer Inkubationsphase von 90 Minuten dargestellt. Der berechnete SI von 0,066 zeigt deutlich, dass die Mischung bestehend aus Menthyllactat und Dihydroavenanthramid D eine stark synergistische Wirkstoffkombination darstellt.

**Tabelle 1: Berechnung des Synergie-Index (SI) eines Menthyl lactat/Dihydroavenanthramid D - Gemisches (Produkt C) bestehend aus dem Vergleichs-Kühlwirkstoff (A) und dem Vergleichs Juckreiz-lindernden Wirkstoff Dihydroavenanthramid D (Produkt B)**

| | A | B | C |
|---|---|---|---|
| | Menthyllactat (Frescolate ML) 1 Gew.-% | Dihydroavenanthramid D 0,05 Gew.-% | Menthyllactat (0,5 Gew.-%) + Dihydroaven-Anthramid D (0,025 Gew.-%) |
| Juckreizlinderung (Intensitätsskala: 0 - 2) 2 = starker Juckreiz 0 = schwacher Juckreiz | 0,5 | 0,6 | 0,3 |
| | | | |
| Kull's Gleichung: SI = CxD/A + CxE/B | | | |
| | | | |
| Juckreizlinderung Produkt A | 0,5 | | |
| Juckreizlinderung Produkt B | 0,6 | | |
| Juckreizlinderung Produkt C | 0,3 | | |
| D: Anteil von A in C | 0,5 | | |
| E: Anteil von B in C | 0,5 | | |
| **SI: Synergie Index** | **0,55** | | |
| | | | |
| Literatur : Synergie Index: | | | |
| D.C.Steinberg; Cosmetics & Toiletries 115 (11); p. 59-62 (2000) | | | |
| F.C.Kull et al.; Applied Microbiology 9; p. 538-541 (1961) | | | |

## Patentansprüche

1. Verwendung einer Verbindung der Formel 1 oder einer Mischung aus zwei oder mehreren unterschiedlichen Verbindungen der Formel 1 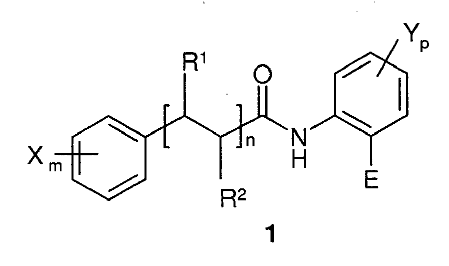 wobei für die Verbindung der Formel 1 oder jede Verbindung der Formel 1 in der Mischung gilt:
m = 0, 1, 2 oder 3,
p = 0, 1 oder 2,
n = 0, 1 oder 2,
wobei bei n = 1 oder 2 jeweils paarweise R¹ und R² jeweils H oder zusammen eine weitere chemische Bindung bedeuten,
wobei bei m = 1, 2 oder 3 jedes X, unabhängig von den anderen, OH, OAlkyl oder OAcyl bedeutet,
wobei bei p = 1 oder 2 jedes Y, unabhängig von den anderen, OH, OAlkyl oder OAcyl bedeutet,
wobei E = H oder ein Rest -COOR³ bedeutet,
R³ = H oder Alkyl, wobei R³ = H auch für die korrespondierenden pharmazeutisch akzeptablen Salze und Solvate steht,
zur Herstellung eines antimikrobiell wirkenden Mittels,
wobei das Mittel ein Mittel (i) zur Hemmung und/oder Verhinderung des Wachstums und/oder zur Abtötung von Mundgeruch verursachenden Organismen und/oder (ii) zur Bekämpfung oder Vermeidung von Mundgeruch ist.

2. Verwendung gemäß Anspruch 1, wobei die Mundgeruch verursachenden Mikroorganismen ausgewählt sind aus der Gruppe bestehend aus: Eubacterium-, Fusobacterium-, Haemophilus-, Neisseria-, Porphyromonas-, Prevotella-, Treponema- und Veillonella-Arten.

3. Verwendung gemäß Anspruch 1 oder 2, wobei für die oder eine der Verbindungen der Formel 1 gilt:
n = 1 oder 2 und die Summe p + m > 0
und/oder p + m > 0 und mindestens einmal ist X oder Y aus der Gruppe ausgewählt, die aus OH und OAcyl besteht.

4. Verwendung gemäß einem der Ansprüche 1 bis 3, wobei für die oder eine der Verbindungen der Formel 1 gilt:
n=1,
p + m ≥ 2,
mit der Maßgabe, dass X und Y zusammen zumindest zweimal aus der Gruppe gewählt sind, die aus OH und OAcyl besteht.

5. Verwendung gemäß Anspruch 1 oder 2, wobei für die oder eine der Verbindungen der Formel 1 gilt:
n=1,
m = 1, 2 oder 3,
mit der Maßgabe, dass X zumindest einmal aus der Gruppe gewählt ist, die aus OH oder OAcyl besteht
und/oder
p = 1 oder 2,
mit der Maßgabe, dass Y zumindest einmal ausgewählt aus der Gruppe ist, die aus OH und OAcyl besteht.

6. Verwendung nach einem der vorangehenden Ansprüche, wobei für die oder eine der Verbindungen der Formel 1 gilt:
n = 1
und
R¹ und R² bedeuten jeweils H oder zusammen eine weitere chemische Bindung.

7. Verwendung gemäß einem der Ansprüche 1 bis 3, wobei die oder eine der Verbindungen der Formel 1 aus der Gruppe ausgewählt ist die besteht aus:

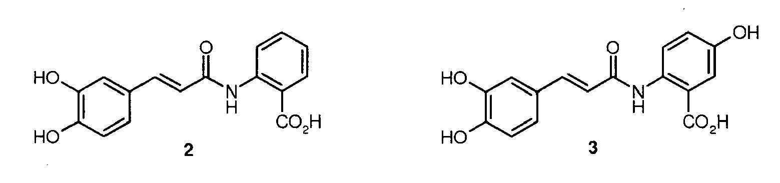

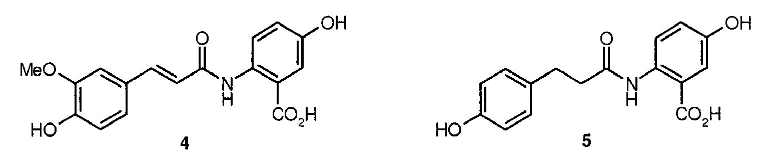

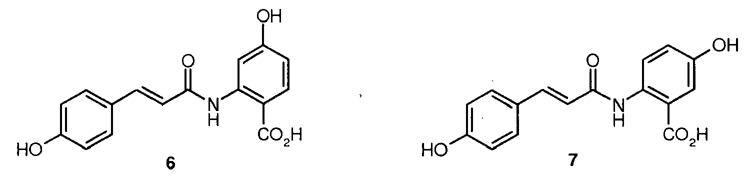

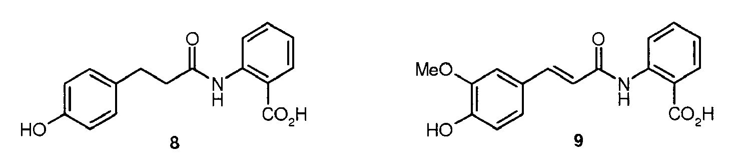

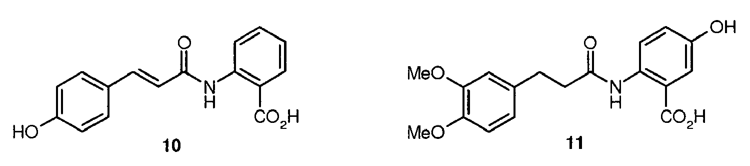

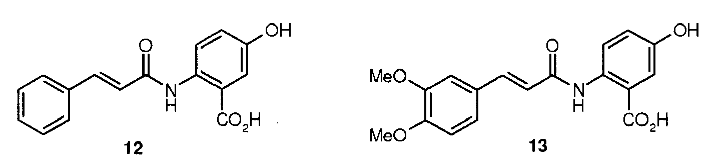

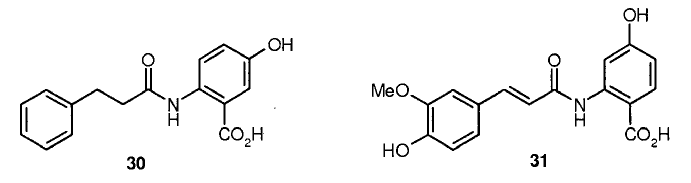

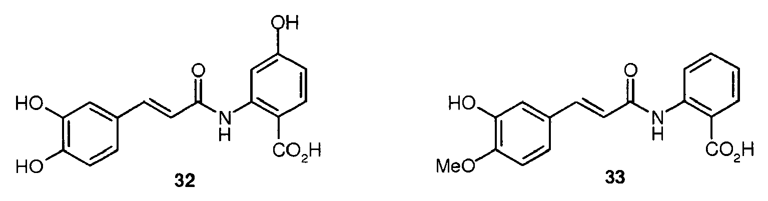

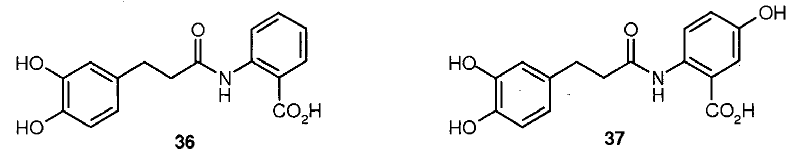

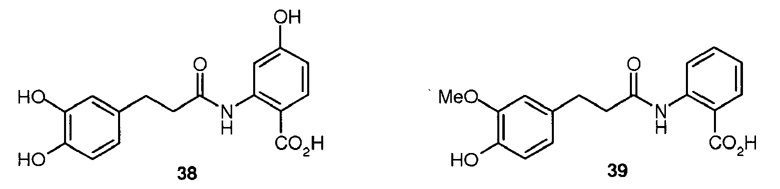

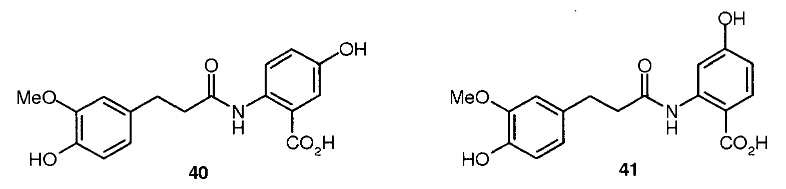

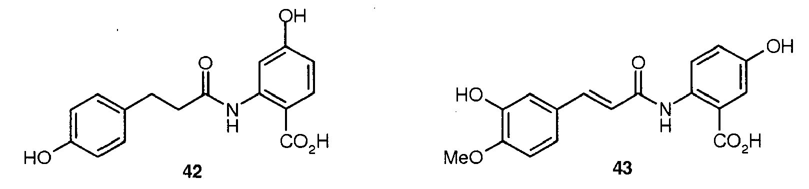

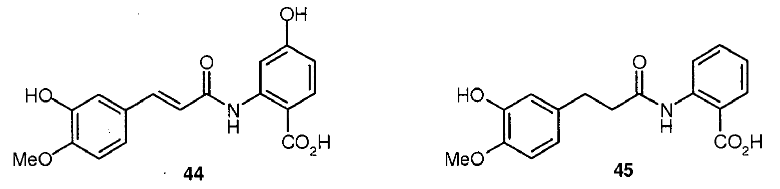

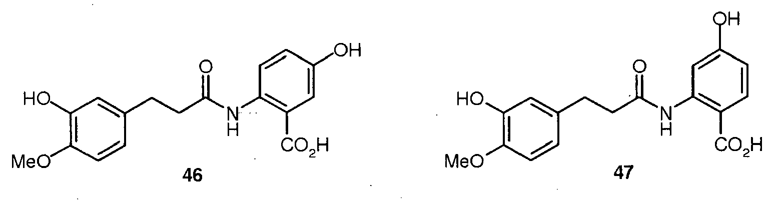

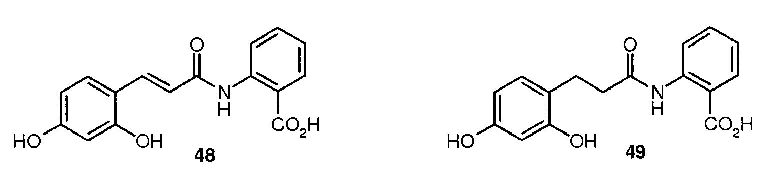

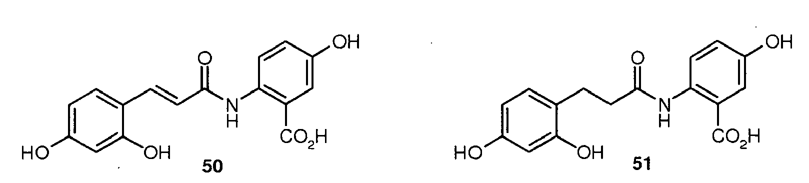

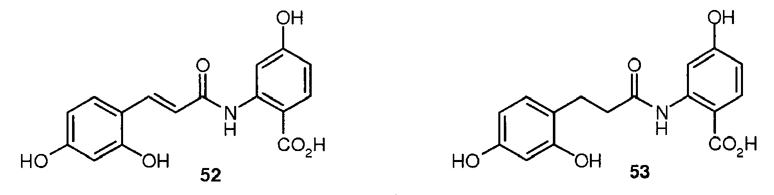

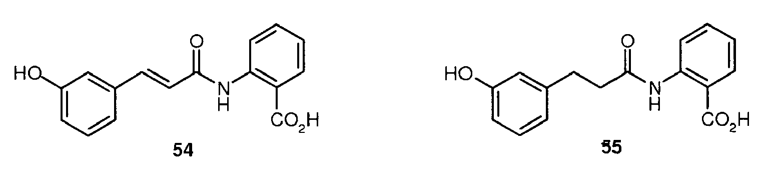

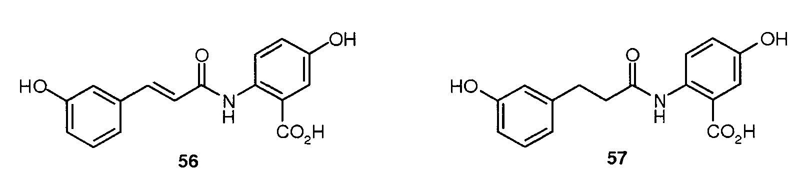

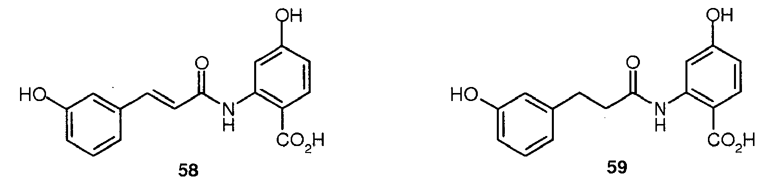

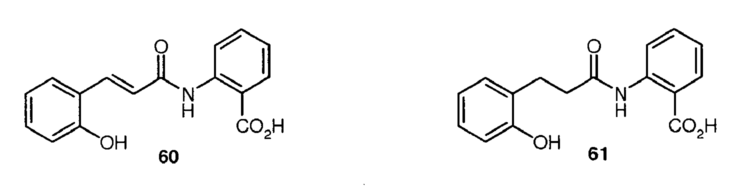

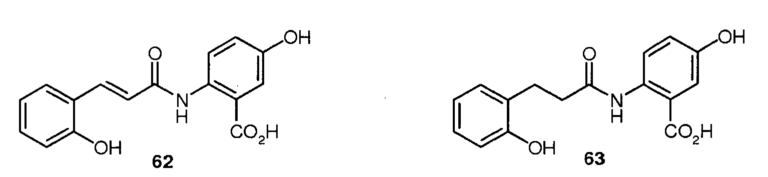

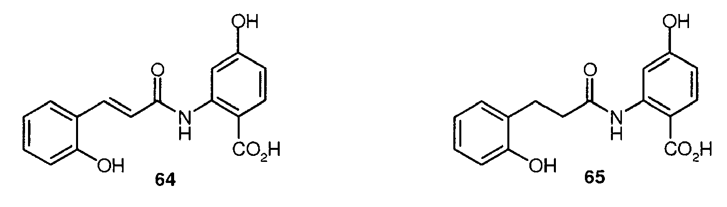

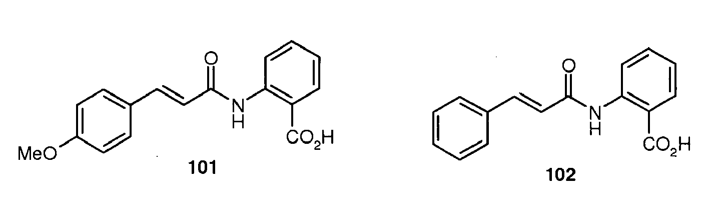

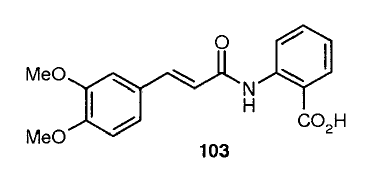

8. Verwendung gemäß Anspruch 1 oder 2, wobei für die oder eine der Verbindungen der Formel 1 gilt:
n = 0.

9. Verwendung gemäß Anspruch 8, wobei für die oder eine der Verbindungen der Formel 1 gilt:
m + p > 2,
mit der Maßgabe, dass zumindest zwei der Substituenten X und Y aus der Gruppe ausgewählt sind, die aus OH und OAcyl besteht.

10. Verwendung gemäß Anspruch 8 oder 9, wobei die oder eine der Verbindungen der Formel 1 aus der Gruppe gewählt ist die besteht aus:

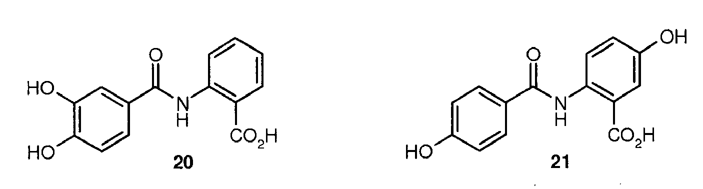

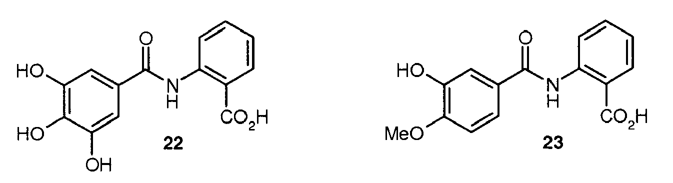

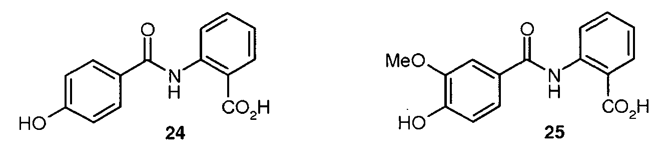

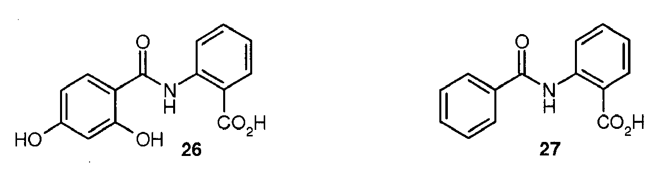

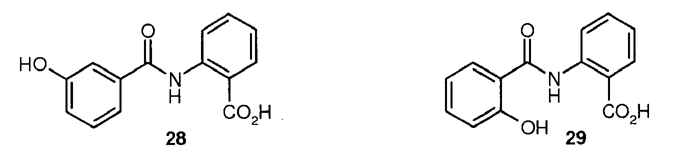

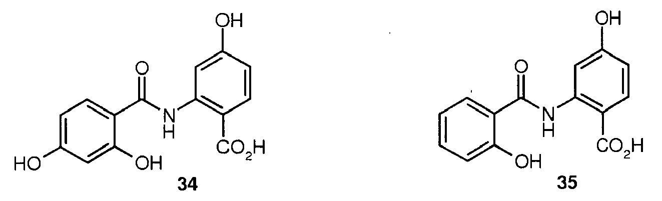

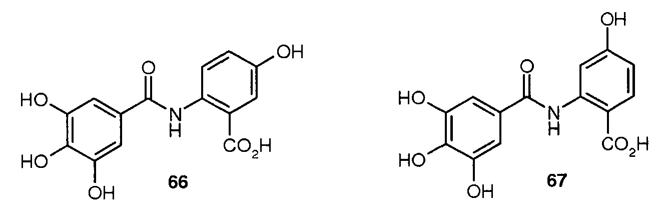

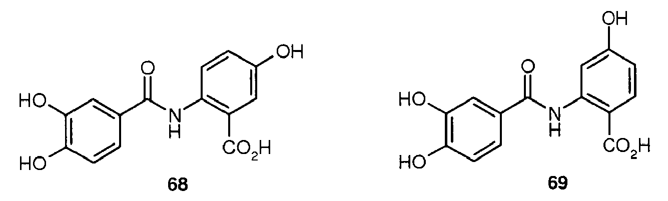

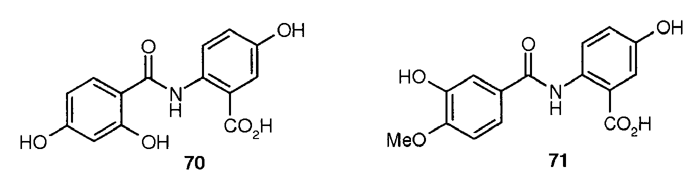

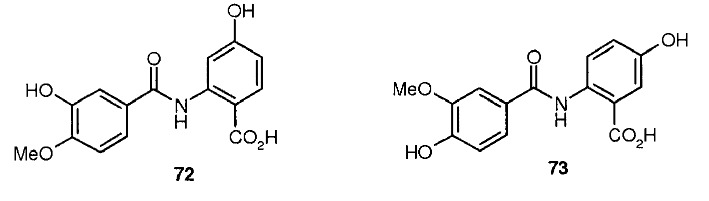

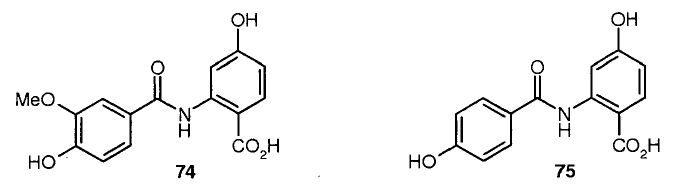

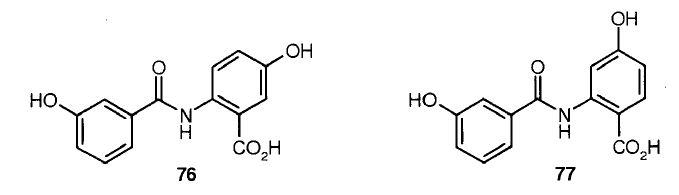

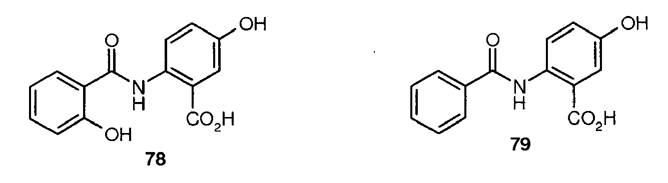

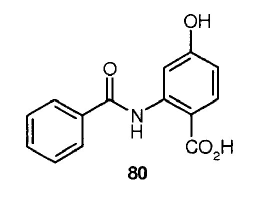

11. Verwendung gemäß Anspruch 8, wobei für die oder eine der Verbindungen der Formel 1 gilt:
n = 0,
m = 1,
p = 0,
X = OH und
E = H.

12. Verwendung gemäß Anspruch 11, wobei die oder eine der Verbindungen Formel 100 ist:

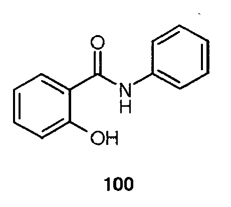

13. Verwendung gemäß einem der Ansprüche 1 bis 6, 8 und 9, wobei gilt:
R³ = CH₃ oder lineares oder verzweigtes Alkyl mit 2 bis 30 C-Atomen.

14. Mundhygieneprodukt umfassend eine Verbindung der Formel 1 oder eine Mischung umfassend zwei oder mehrere Verbindungen der Formel 1 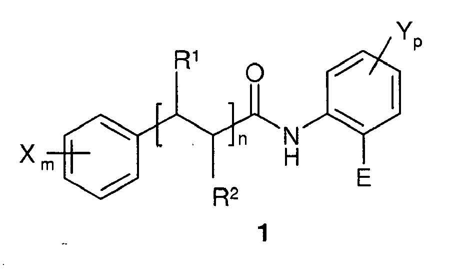 wobei für die Verbindung der Formel 1 oder jede Verbindung der Formel 1 in der Mischung gilt:
m = 0, 1, 2 oder 3,
p = 0, 1 oder 2,
n = 0, 1 oder 2,
wobei bei n = 1 oder 2 jeweils paarweise R¹ und R² jeweils H oder zusammen eine weitere chemische Bindung bedeuten,
wobei bei m = 1, 2 oder 3 jedes X, unabhängig von den anderen, OH, OAlkyl oder OAcyl bedeutet,
wobei bei p = 1 oder 2 jedes Y, unabhängig von den anderen, OH, OAlkyl oder OAcyl bedeutet,
wobei E = H oder einen Rest -COOR³ bedeutet,
R³ = H oder Alkyl, wobei R³ = H auch für die korrespondierenden pharmazeutisch akzeptablen Salze und Solvate steht,
in einer zur Bekämpfung und/oder Vermeidung von Mundgeruch ausreichenden Menge,
wobei das Produkt ausgewählt ist aus der Gruppe bestehend aus Zahncremes, Zahnpasten, Zahngele, Lutschpastillen, Lutschtabletten, Bonbons, Kaugummis, Kaubonbons und zahnpflegende Kaugummis.

15. Mundwasser umfassend eine Mischung umfassend oder bestehend aus:
(a) einer oder mehrerer Verbindungen der Formel 1 A, 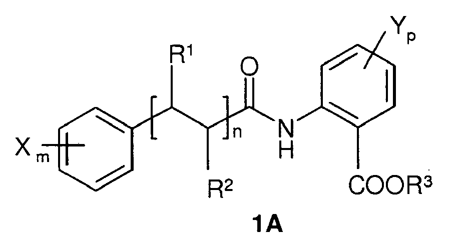 wobei für die Verbindung bzw. jede Verbindung der Formel 1 A gilt:
m = 0, 1, 2 oder 3,
p = 0, 1 oder 2,
n = 0, 1 oder 2,
wobei bei n = 1 oder 2 jeweils paarweise R¹ und R² jeweils H oder zusammen eine weitere chemische Bindung bedeuten;
wobei bei m = 1, 2 oder 3 jedes X, unabhängig von den anderen, OH, OAlkyl oder OAcyl bedeutet,
wobei bei p = 1 oder 2 jedes Y, unabhängig von den anderen, OH, OAlkyl oder OAcyl bedeutet,
R³ = H oder Alkyl, wobei R³ = H auch für die korrespondierenden kosmetisch oder pharmazeutisch akzeptablen Salze und Solvate steht,
und
(b) einem oder mehreren Kühlwirkstoffen,
wobei der pH-Wert des Mundwassers pH 6,5 bis 8 beträgt.

## Claims

1. Use of a compound of formula 1 or of a mixture of two or more different compounds of formula 1 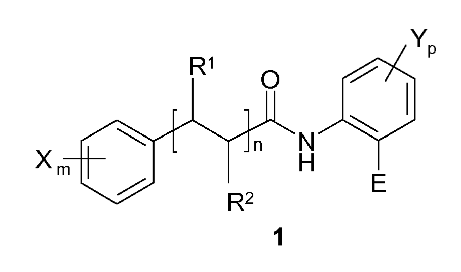 wherein for the compound of formula 1 or each compound of formula 1 in the mixture the following applies:
m=0, 1, 2 or 3,
p = 0, 1 or 2,
n = 0, 1 or 2,
wherein if n = 1 or 2, R¹ and R² in each case pairwise both denote H or together denote a further chemical bond,
wherein if m = 1, 2 or 3 each X, independent of the others, denotes OH, Oalkyl or Oacyl,
wherein if p = 1 or 2 each Y, independent of the others, denotes OH, Oalkyl or Oacyl,
wherein E = H or denotes a moiety -COOR³,
R³ = H or alkyl, wherein R³ = H also stands for the corresponding pharmaceutically acceptable salts and solvates,
for the production of an antimicrobially active agent,
wherein the agent is an agent (i) for the inhibition and/or prevention of growth and/or for killing organisms which cause oral malodor and/or (ii) for fighting or avoiding oral malodor.

2. Use according to claim 1, wherein the microorganisms which cause oral malodor are selected from the group consisting of: eubacterium, fusobacterium, haemophilus, neisseria, porphyromonas, prevotella, treponema and veillonella species.

3. Use according to claim 1 or 2, wherein for the or one of the compound(s) of formula 1 the following applies:
n = 1 or 2 and the sum p + m > 0
and/or p + m > 0 and X or Y is at least once selected from the group which consists of OH and Oacyl.

4. Use according to one of claims 1 to 3, wherein for the or one of the compound(s) of formula 1 the following applies:
n = 1,
p + m ≥ 2,
with the proviso that X and Y together are at least twice selected from the group which consists of OH and Oacyl.

5. Use according to claim 1 or 2, wherein for the or one of the compound(s) of formula 1 the following applies:
n = 1,
m = 1, 2 or 3,
with the proviso that X is at least once selected from the group which consists of OH and Oacyl
and/or
p = 1 or 2,
with the proviso that Y is at least once selected from the group which consists of OH and Oacyl.

6. Use according to one of the preceding claims, wherein for the or one of the compound(s) of formula 1 the following applies:
n = 1
and
R¹ and R² each denote H or together a further chemical bond.

7. Use according to one of claims 1 to 3, wherein the or one of the compound(s) of formula 1 is selected from the group which consists of:

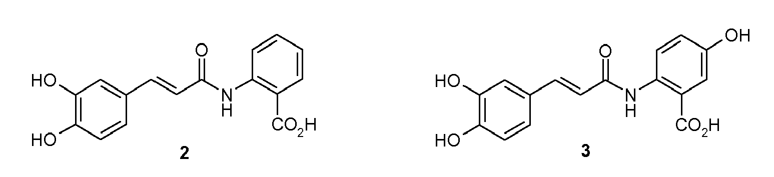

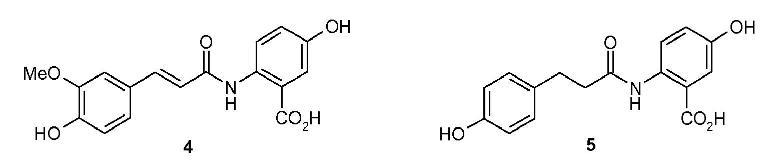

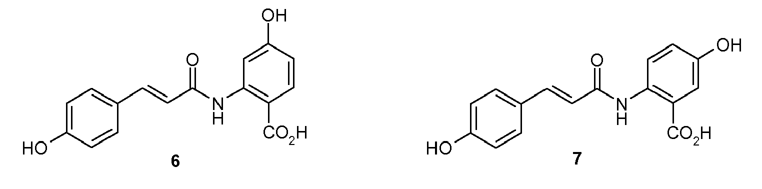

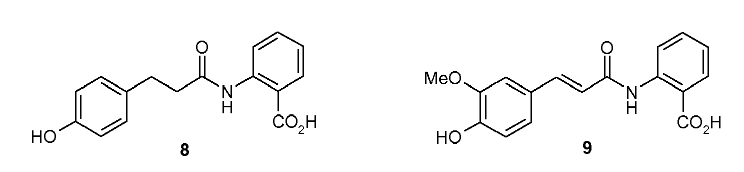

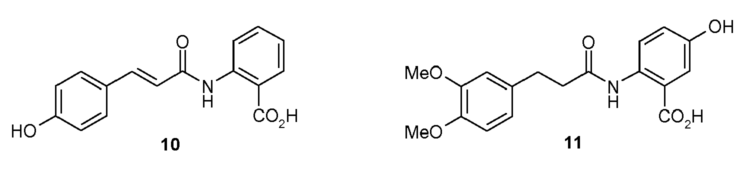

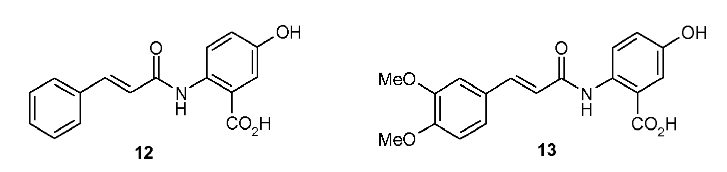

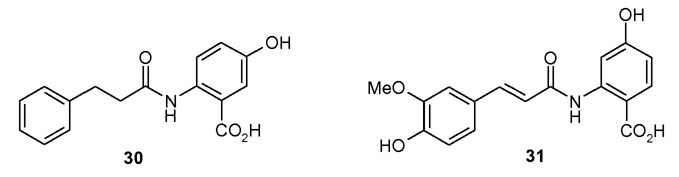

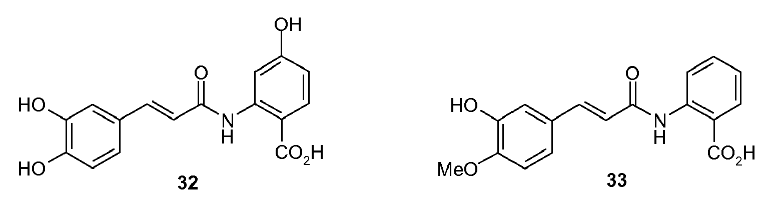

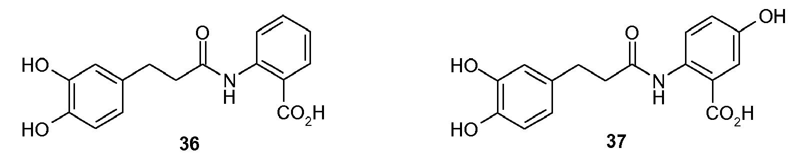

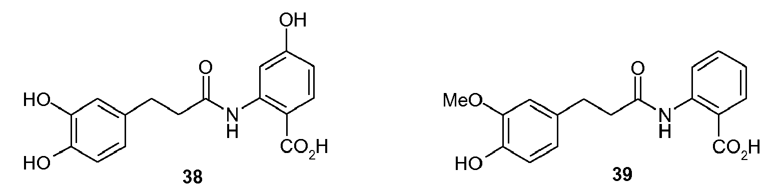

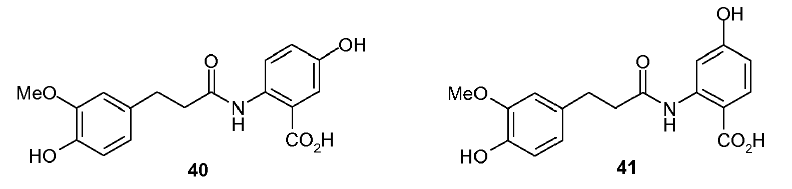

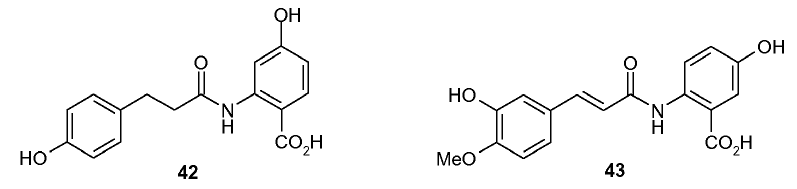

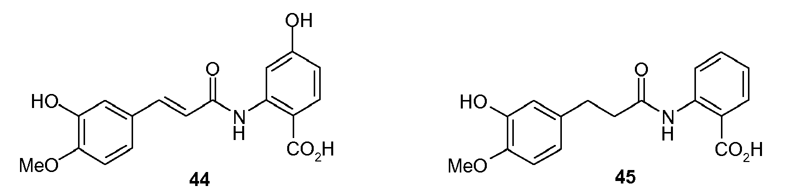

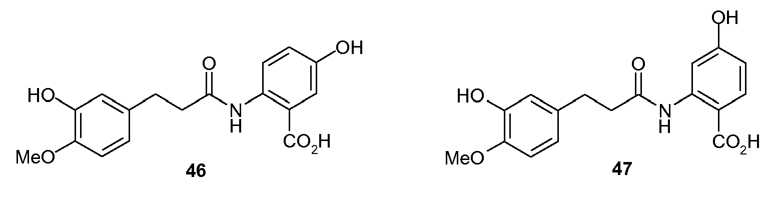

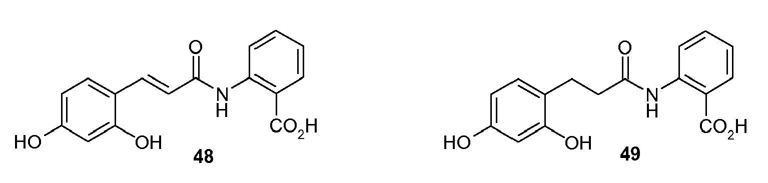

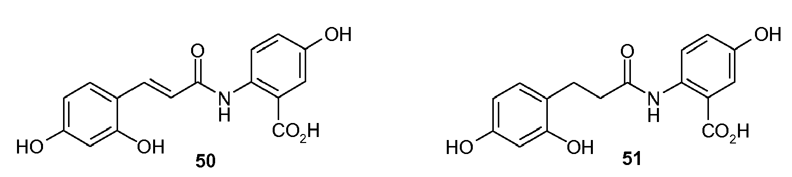

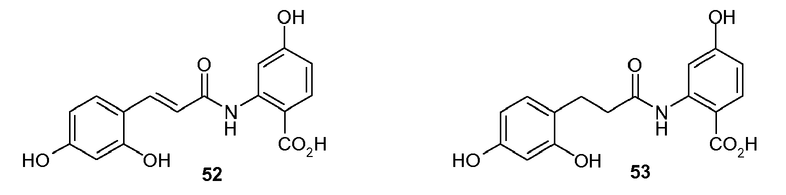

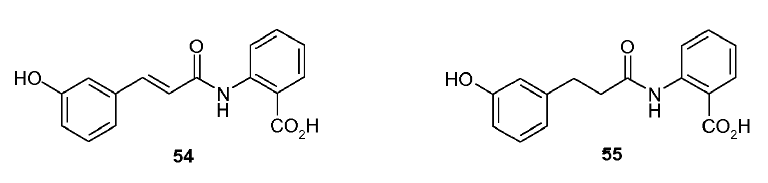

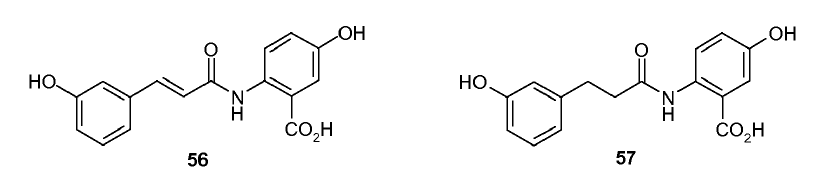

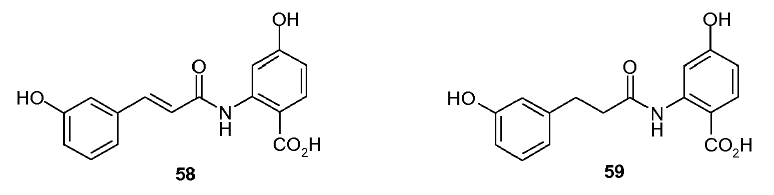

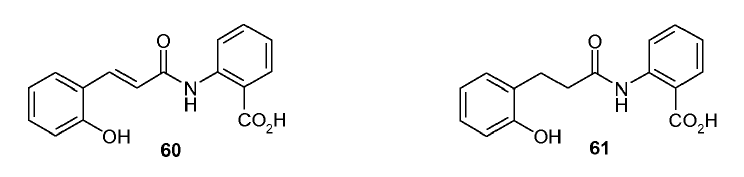

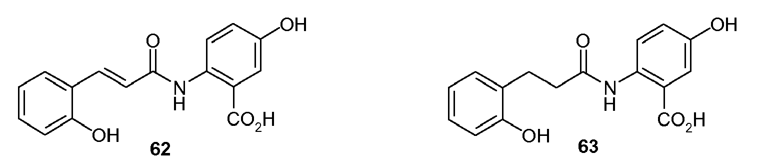

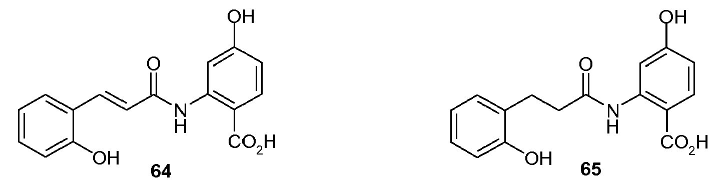

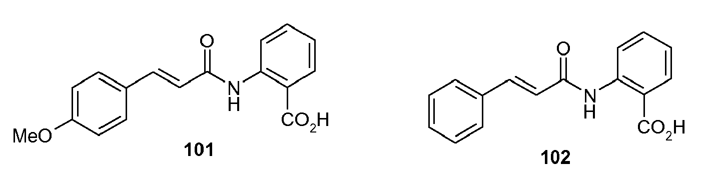

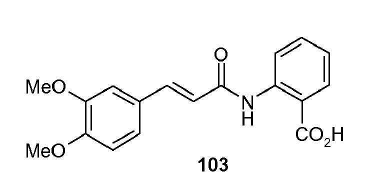

8. Use according to claim 1 or 2, wherein for the or one of the compound(s) of formula 1 the following applies:
n=0.

9. Use according to claim 8, wherein for the or one of the compound(s) of formula 1 the following applies:
m + p > 2,
with the proviso that at least two of the substituents X and Y are selected from the group which consists of OH and Oacyl.

10. Use according to claim 8 or 9, wherein the or one of the compound(s) of formula 1 is selected from the group which consists of:

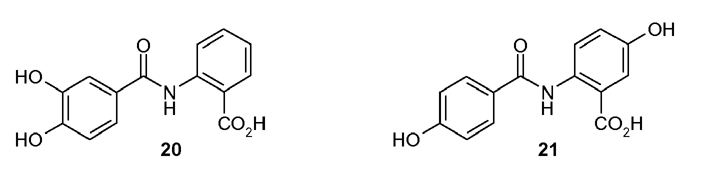

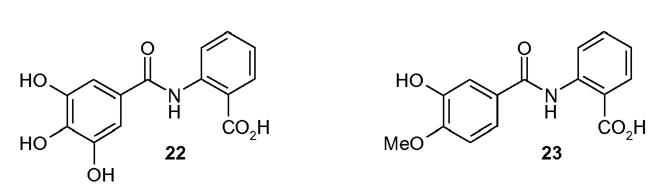

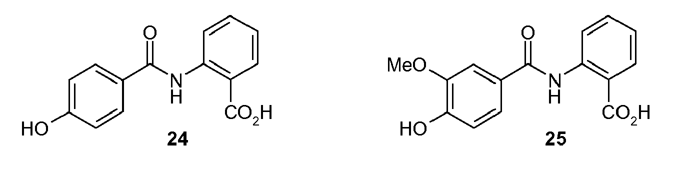

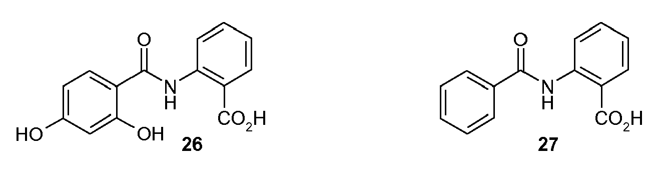

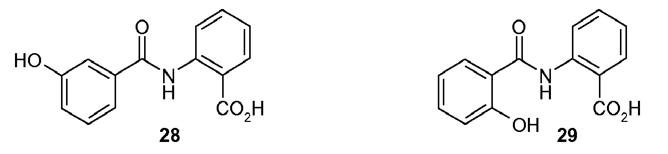

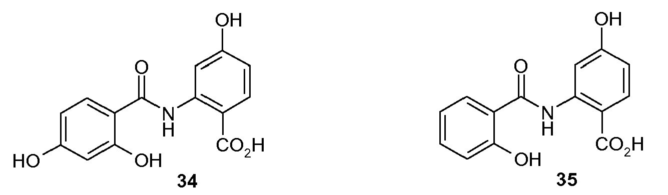

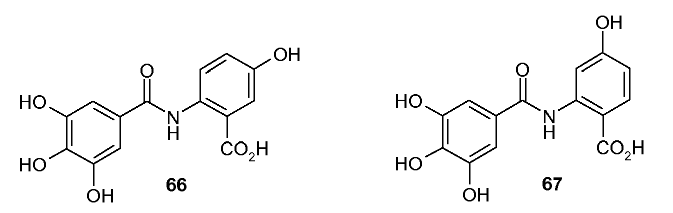

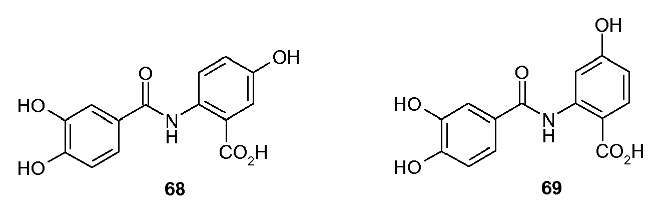

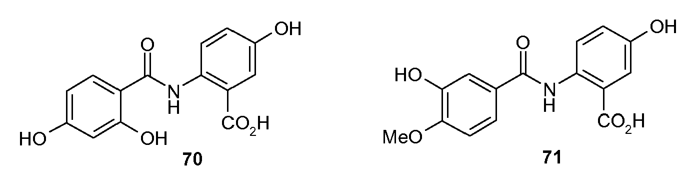

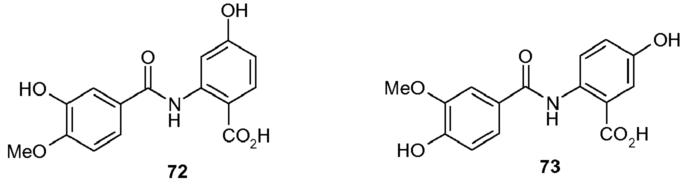

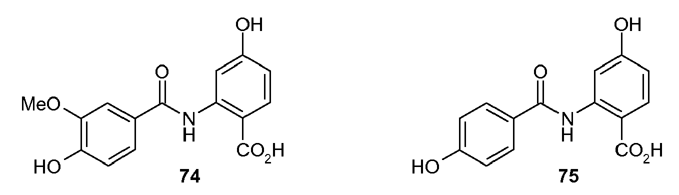

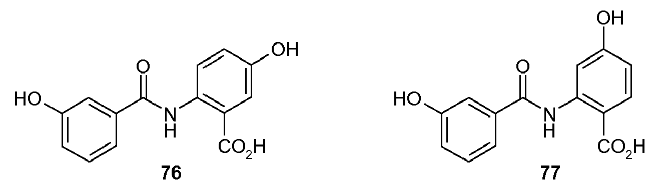

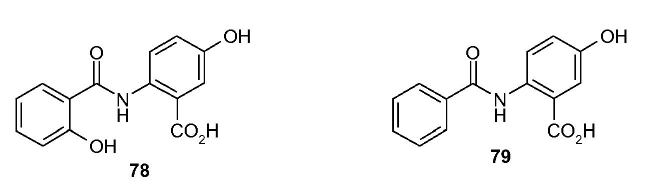

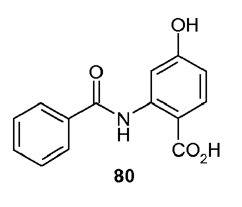

11. Use according to claim 8, wherein for the or one of the compound(s) of formula 1 the following applies:
n = 0,
m = 1,
p = 0,
X = OH and
E = H.

12. Use according to claim 11, wherein die the or one of the compound(s) is formula 100:

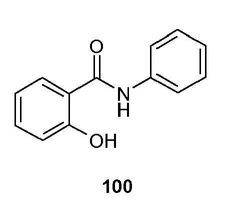

13. Use according to one of claims 1 to 6, 8 and 9, wherein the following applies:
R³ = CH₃ or linear or branched alkyl with 2 to 30 C-atoms.

14. Oral hygiene product comprising a compound of formula 1 or a mixture comprising two or more compounds of formula 1 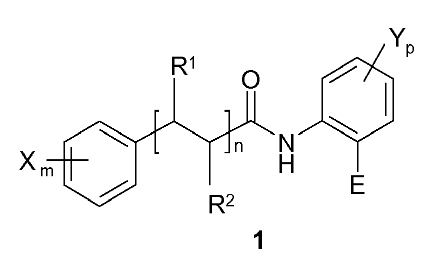 wherein for the compound of formula 1 or each compound of formula 1 in the mixture the following applies:
m=0, 1, 2 or 3,
p = 0, 1 or 2,
n = 0, 1 or 2,
wherein if n = 1 or 2, R¹ and R² in each case pairwise both denote H or together denote a further chemical bond,
wherein if m = 1, 2 or 3 each X, independent of the others, denotes OH, Oalkyl or Oacyl,
wherein if p = 1 or 2 each Y, independent of the others, denotes OH, Oalkyl or Oacyl,
wherein E = H or denotes a moiety -COOR³,
R³ = H or alkyl, wherein R³ = H also stands for the corresponding pharmaceutically acceptable salts and solvates,
in an amount sufficient for fighting and/or avoiding oral malodor,
wherein the product is selected from the group consisting of tooth crèmes, tooth pastes, tooth gels, lozenges, bonbons, chewing gums, chews and toothcare chewing gums.

15. Mouthwash comprising a mixture comprising or consisting of:
(a) one or more compound(s) of formula 1A, 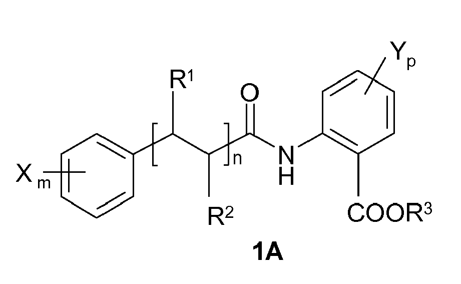 wherein for the compound or each compound of formula 1A, respectively, the following applies:
m = 0, 1, 2 or 3,
p = 0, 1 or 2,
n = 0, 1 or 2,
wherein if n = 1 or 2, R¹ and R² in each case pairwise both denote H or together denote a further chemical bond;
wherein if m = 1, 2 or 3 each X, independent of the others, denotes OH, Oalkyl or Oacyl,
wherein if p = 1 or 2 each Y, independent of the others, denotes OH, Oalkyl or Oacyl,
R³ = H or alkyl, wherein R³ = H also stands for the corresponding pharmaceutically acceptable salts and solvates,
and
(b) one or more cooling agents,
wherein the pH value of the mouthwash is pH 6.5 to 8.

## Revendications

1. Utilisation d'un composé de la formule 1 ou d'un mélange de deux ou plusieurs composés différents de la formule 1 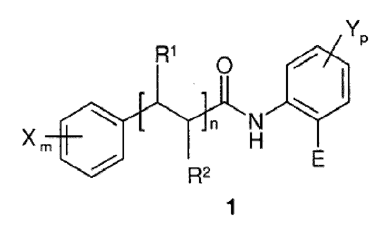 pour le composé de la formule 1 ou pour chaque composé de la formule 1 dans le mélange valant :
m = 0, 1, 2 ou 3,
p = 0, 1 ou 2,
n = 0, 1 ou 2,
où, si n = 1 ou 2, alors R¹ und R² signifient, respectivement par paires, chacun H ou ensemble une autre liaison chimique,
où, si m = 1, 2 ou 3, alors chaque X, indépendamment des autres, signifie OH, O-alkyle ou O-acyle,
où, si p = 1 ou 2, alors chaque Y, indépendamment des autres, signifie OH, O-alkyle ou O-acyle,
où E est H ou signifie un radical -COOR³,
R³ = H ou alkyle, où R³ = H désigne également les sels et solvates pharmaceutiquement acceptables correspondants,
pour la préparation d'un agent agissant de manière antimicrobienne,
dans laquelle ledit agent est un agent (i) destiné à inhiber et/ou à empêcher la croissance de et/ou à détruire des organismes qui causent la mauvaise haleine,
et/ou (ii) destiné à lutter contre ou à éviter la mauvaise haleine.

2. Utilisation selon la revendication 1, dans laquelle les micro-organismes qui causent la mauvaise haleine sont choisis dans le groupe constitué par : les genres Eubacterium, Fusobacterium, Haemophilus, Neisseria, Porphyromonas, Prevotella, Treponema et Veillonella.

3. Utilisation selon la revendication 1 ou 2, pour le ou l'un des composé(s) de la formule 1 valant :
n = 1 ou 2 et la somme p + m > 0
et/ou p + m > 0 et X ou Y est choisi au moins une fois dans le groupe qui est constitué par OH et O-acyle.

4. Utilisation selon l'une quelconque des revendications 1 à 3, pour le ou l'un des composé(s) de la formule 1 valant :
n = 1
p + m ≥ 2,
sous réserve que X et Y soient choisis ensemble, au moins deux fois, dans le groupe qui est constitué par OH et O-acyle.

5. Utilisation selon la revendication 1 ou 2, pour le ou l'un des composé(s) de la formule 1 valant :
n = 1
m = 1, 2 ou 3,
sous réserve que X soit choisi, au moins une fois, dans le groupe qui est constitué par OH ou O-acyle
et/ou
p = 1 ou 2,
sous réserve que Y soit choisi, au moins une fois, dans le groupe qui est constitué par OH et O-acyle.

6. Utilisation selon l'une quelconque des revendications précédentes, pour le ou l'un des composé(s) de la formule 1 valant :
n = 1
et
R¹ et R² signifient chacun H ou ensemble une autre liaison chimique.

7. Utilisation selon l'une quelconque des revendications 1 à 3, dans laquelle le ou l'un des composé(s) de la formule 1 est choisi dans le groupe qui est constitué par:

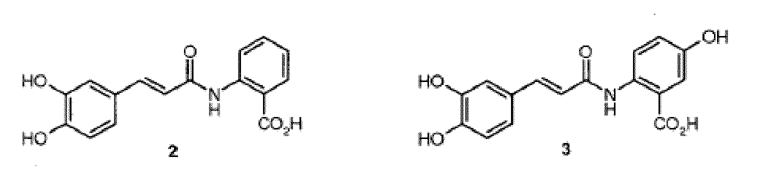

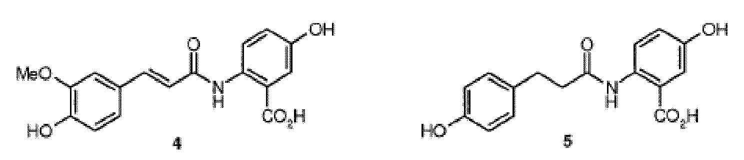

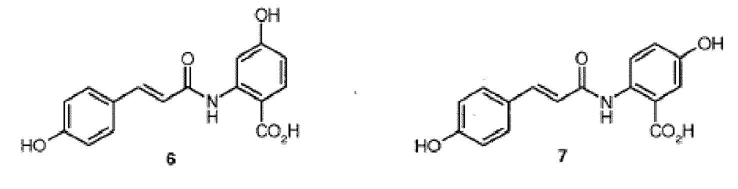

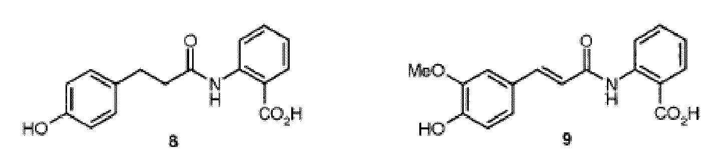

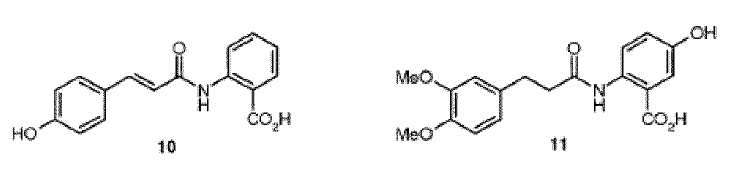

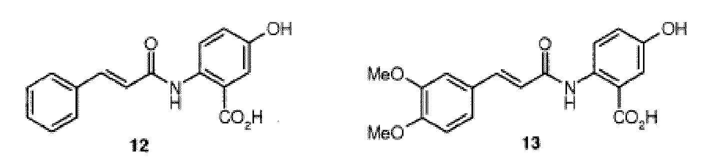

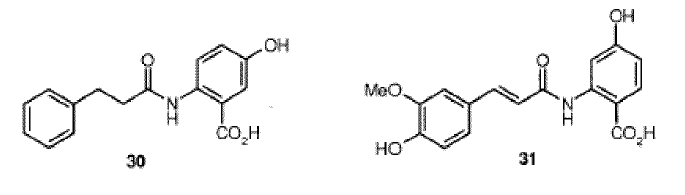

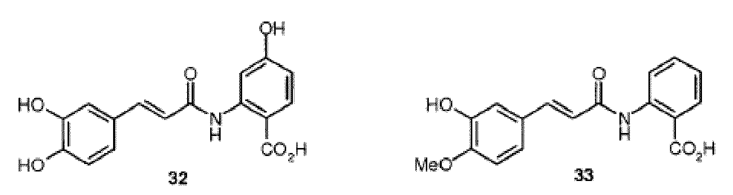

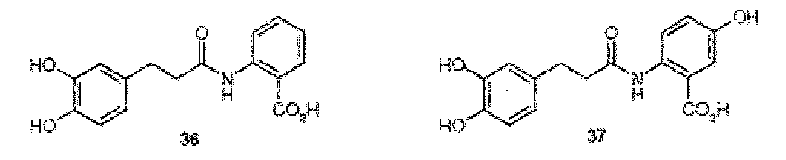

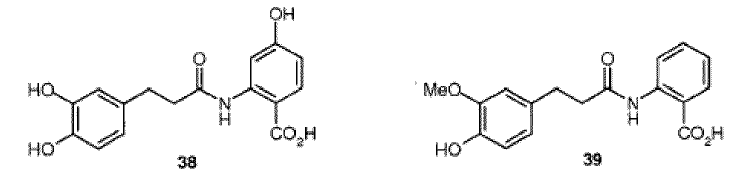

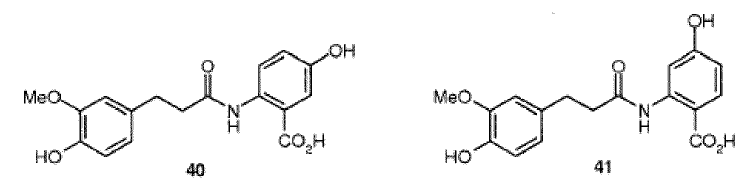

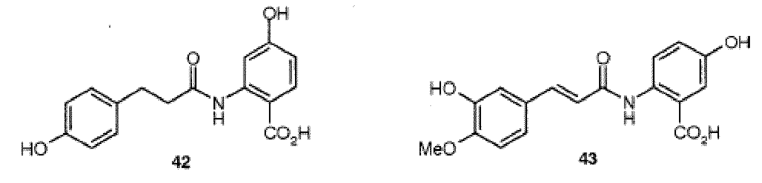

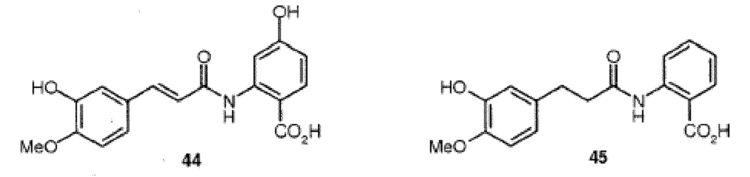

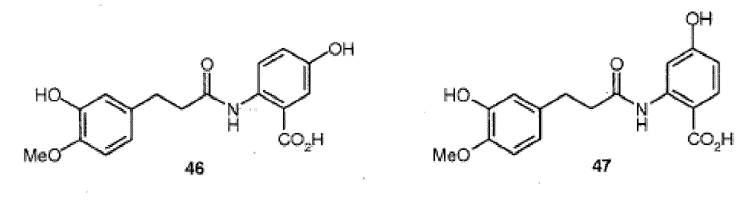

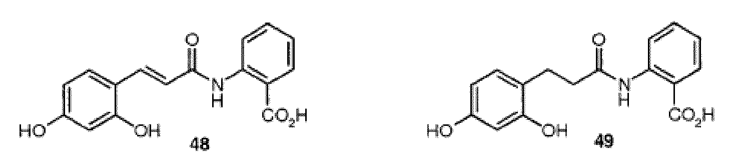

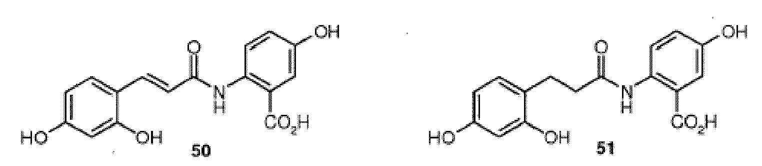

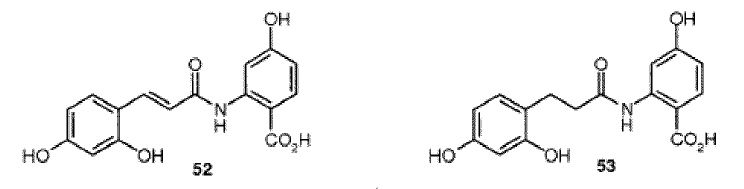

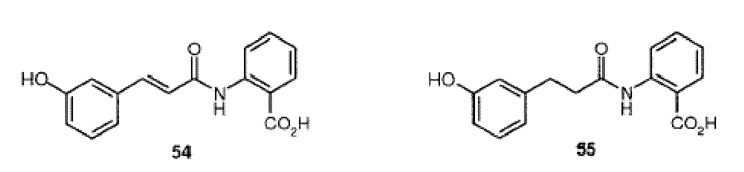

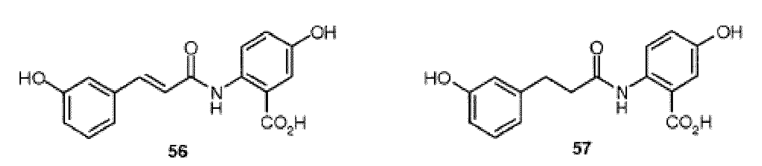

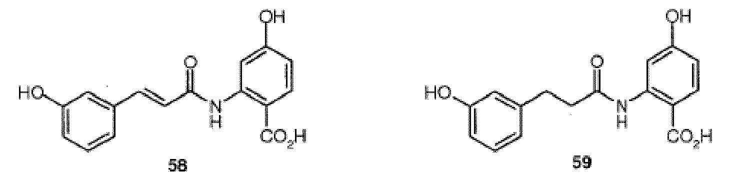

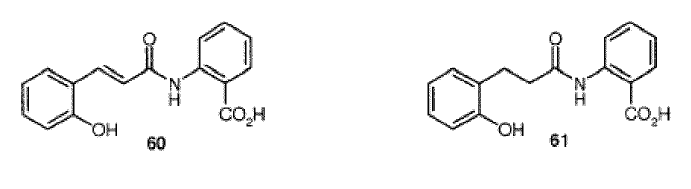

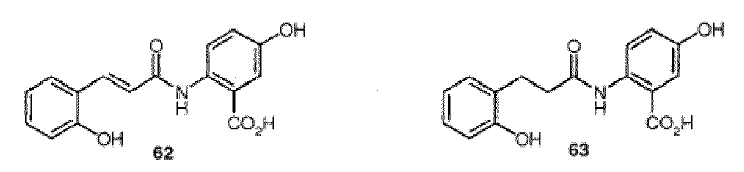

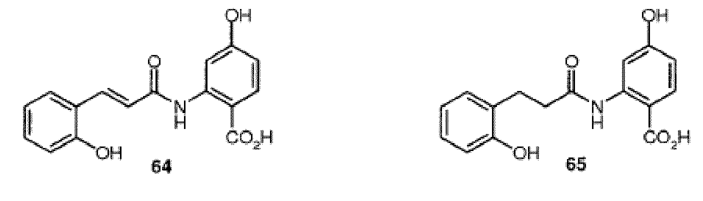

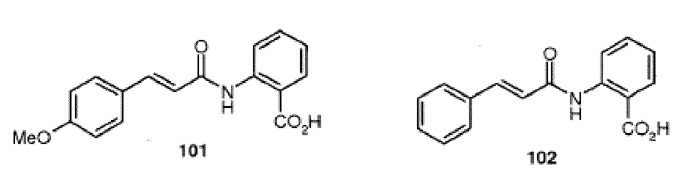

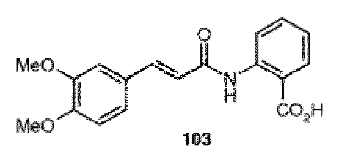

8. Utilisation selon la revendication 1 ou 2, pour le ou l'un des composé(s) de la formule 1 valant :
n=0.

9. Utilisation selon la revendication 8, pour le ou l'un des composé(s) de la formule 1 valant :
m + p > 2,
sous réserve que deux au moins des substituants X et Y soient choisis dans le groupe qui est constitué par OH et O-acyle.

10. Utilisation selon la revendication 8 ou 9, dans laquelle le ou l'un des composé(s) de la formule 1 est choisi dans le groupe qui est constitué par :

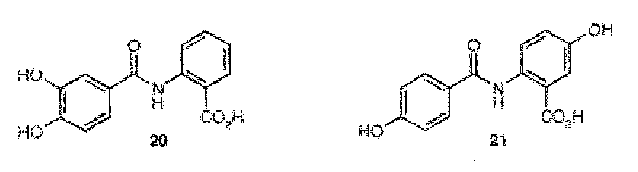

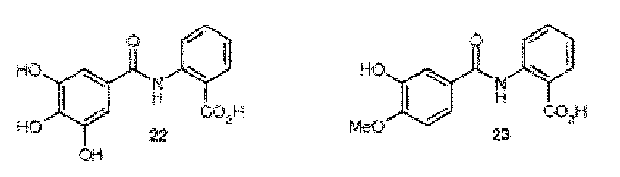

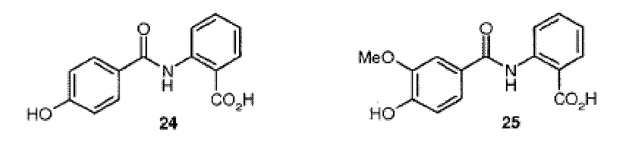

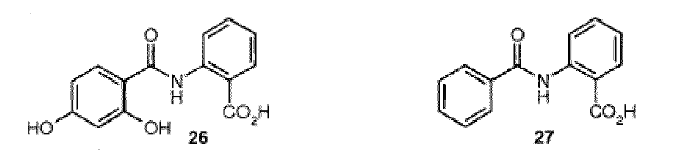

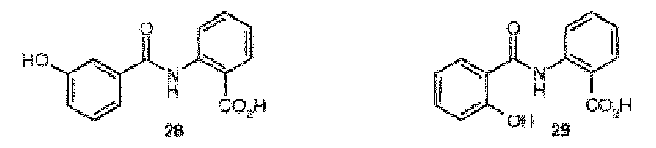

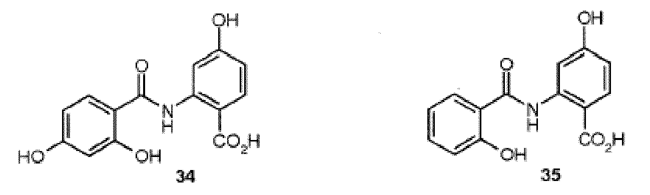

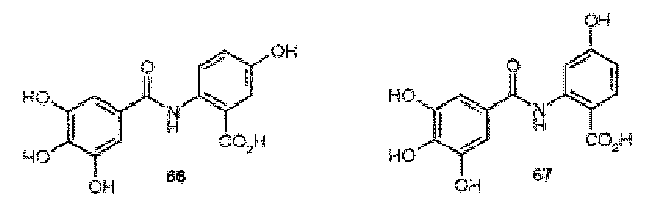

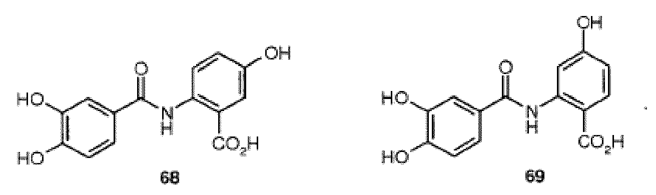

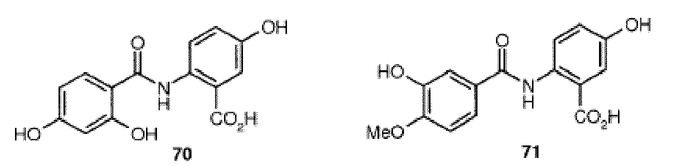

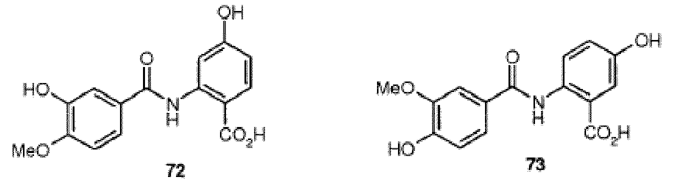

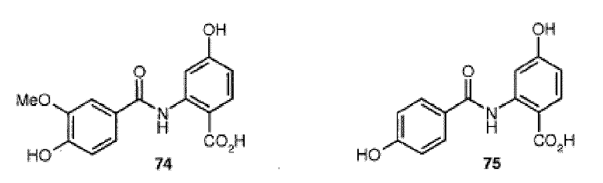

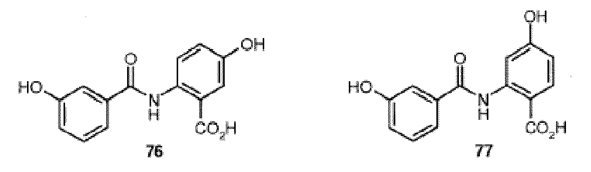

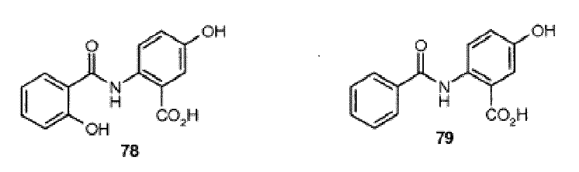

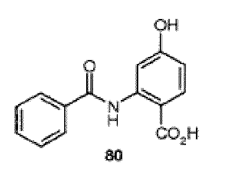

11. Utilisation selon la revendication 8, pour le ou l'un des composé(s) de la formule 1 valant :
n = 0,
m=1,
p = 0,
X = OH et
E = H.

12. Utilisation selon la revendication 11, dans laquelle le ou l'un des composé(s) est la formule 100 :

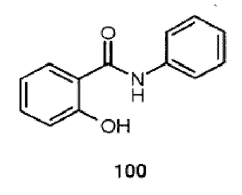

13. Utilisation selon l'une quelconque des revendications 1 à 6, 8 et 9, où :
R³ = CH₃ ou un alkyle linéaire ou ramifié ayant 2 à 30 atomes de carbone.

14. Produit d'hygiène bucco-dentaire comprenant un composé de la formule 1 ou un mélange comprenant deux ou plusieurs composés de la formule 1 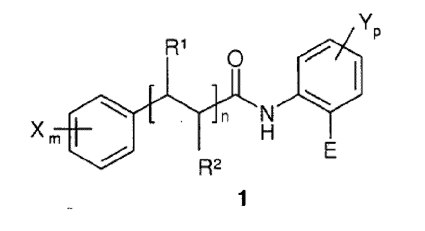 pour le composé de la formule 1 ou pour chaque composé de la formule 1 dans le mélange valant :
m = 0, 1, 2 ou 3,
p = 0, 1 ou 2,
n = 0, 1 ou 2,
où, si n = 1 ou 2, alors R¹ und R² signifient, respectivement par paires, chacun H ou ensemble une autre liaison chimique,
où, si m = 1, 2 ou 3, alors chaque X, indépendamment des autres, signifie OH, O-alkyle ou O-acyle,
où, si p = 1 ou 2, alors chaque Y, indépendamment des autres, signifie OH, O-alkyle ou O-acyle,
où E est H ou signifie un radical -COOR³,
R³ = H ou alkyle, où R³ = H désigne également les sels et solvates pharmaceutiquement acceptables correspondants,
en une quantité suffisante pour lutter contre et/ou pour éviter la mauvaise haleine,
ledit produit étant choisi dans le groupe constitué par les dentifrices, les pâtes dentifrices, les gels dentifrices, les pastilles à sucer, les comprimés à sucer, les bonbons, les gommes à mâcher, les bonbons à mâcher et les gommes à mâcher pour soins dentaires.

15. Eau buccale comprenant un mélange comprenant ou se composant de :
(a) un ou plusieurs composé(s) de la formule 1A, 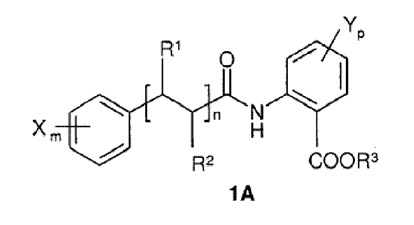 pour le composé ou bien pour chaque composé de la formule 1A valant :
m = 0, 1, 2 ou 3,
p = 0, 1 ou 2,
n = 0, 1 ou 2,
où, si n = 1 ou 2, alors R¹ und R² signifient, respectivement par paires, chacun H ou ensemble une autre liaison chimique,
où, si m = 1, 2 ou 3, alors chaque X, indépendamment des autres, signifie OH, O-alkyle ou O-acyle,
où, si p = 1 ou 2, alors chaque Y, indépendamment des autres, signifie OH, O-alkyle ou O-acyle,
R³ = H ou alkyle, où R³ = H désigne également les sels et solvates cosmétiquement et pharmaceutiquement acceptables correspondants,
et
(b) un ou plusieurs principe(s) actif(s) rafraîchissants(s),
la valeur pH de ladite eau buccale étant comprise entre 6,5 et 8 pH.
